(19) Europäisches Patentamt
European Patent Office
Office européen des brevets

(11) EP 2 058 403 A1

(12) EUROPEAN PATENT APPLICATION

(43) Date of publication:
**13.05.2009 Bulletin 2009/20**

(51) Int Cl.:
***C12Q 1/68*** *(2006.01)*

(21) Application number: **07021849.0**

(22) Date of filing: **09.11.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicants:
- **Rheinische Friedrich-Wilhelms-Universität Bonn**
  **53113 Bonn (DE)**
- **Heinrich-Heine-Universität Düsseldorf**
  **40225 Düsseldorf (DE)**

(72) Inventors:
- **Waha, Andreas**
  **53125 Bonn (DE)**
- **Felsberg, Jörg**
  **41540 Dormagen (DE)**
- **Pietsch, Torsten**
  **53359 Rheinbach (DE)**

(74) Representative: **von Kreisler Selting Werner Patentanwälte**
**P.O. Box 10 22 41**
**50462 Köln (DE)**

# (54) DUSP4 as clinically relevant epigenetic marker and therapeutic target in gliomas and other tumors

(57) The present invention provides a method for diagnosis or prognosis of a cancer and a method for prediction of the responsiveness of certain tumor types, said methods comprising determining the methylation degree, i.e. the detection of methylated CpG sites in of the *DUSP4*/*MKP2* gene/promoter. The invention further provides a method for determination of the methylation degree of the promoter region of the dual-specificity phosphatase 4/map kinase phosphatase 2 (*DUSP4*/*MKP2)* gene, which is suitable for prediction of the responsiveness of certain tumor types and for diagnosis or prognosis of cancer. Amplification products and primers which may be used in said method and kits for performing the method are also provided.

EP 2 058 403 A1

SEQUENCE LISTING

<110> Rheinische Friedrich-Wilhelms-Universitaet Bonn
Heinrich-Heine-Universitaet Duesseldorf

<120> DUSP4 as Clinically Relevant Epigenetic Marker and Therapeutic Target in Gliomas and Other Tumors

<130> 072716

<160> 8

<170> PatentIn version 3.3

<210> 1
<211> 9050
<212> DNA
<213> Homo sapiens

<400> 1
atataaataa aagacctctc cttgggcaat cccatcccaa ttcctacaaa tatgccttag 60

ttaattaaat taactttgaa atgacattcc gtgtcttgct ccaaattgcc tttgttttct 120

ccaagatctg ctcattgaca agctgtttca caaggttcct gtttaaaatg gttatgtttc 180

gtccacagac tgcagacgaa ttggagacag gttttatcta tagccggcat ggagtagccc 240

tcgcgttgta ctcatttaac tgtggaccaa tggttcacaa acatacaatt aaaaaatata 300

tatctagggt gtttacacag tatcccgata aatacaatat ggtaacttaa atctcctttg 360

ctgaaattca cacaactgaa ggccaattcc tgagttggca atggggtgtg gattttaaaa 420

attagtcttt tttaaaaatc aggccttcgc tttcgcaatt caagtggctc tcggcttaaa 480

cacattaagt cccaagctgt gactcccttc cagtgaacag ccgcgtaggc gcgggcacgc 540

acacgtggac acacacactc acacgcgccc tcccacaccc ctttccgtgt gaatcgcctg 600

cgggatttag ggcttctcca ccagcgatta ctgtcaggtg cagaaagcaa gcctctttgt 660

tctatgggca ttttcctccc cctggatttg actgttattt actggccacg tgagcgccag 720

gcgcgggtcc ggcggggaag gcgaccccag aacagggtag acaaaagctg gcccagagcg 780

cgacccgtgc gccccaggga cgtctcccca gctcctgcga cacgcgtgaa attggcccgg 840

acctgcctcc tgagaggctt ttcccagaaa tgtcttgtta aagcctttgt aaaggaatca 900

gtgtttactt gcgccgggag agctccggcc ctgggtcctg gcaggcggcg gatttctgag 960

ctgctggaga gcaacccggg aaagcaaaat cagccttgga ctgtcggggc ccctggggct 1020

ccttgctcct tatcgaaagc gagttattag atagaccccc acagtcccat tcagcagaat 1080

gggaaactgg actcagacac cttttgccct ccccacaggg aggccacgcc gtgcggcttt 1140

gaccactgat ctcccacccg gagtgcccga cgcccagggt cctgccaccg ggcagcctcg 1200

gtcagtccac agttgtggct ccttccaggg cctggactag ggtgagcaca agccttgagc 1260

gcaacattta agaagggcgc cgaaaagtca gtaatcaaaa gaaatatctt gatgcaatgc 1320

```
ctctttaaaa gaaaaaaaaa tgcaaaaaat ccatgatgaa taaaatacta aattttaaaa   1380
agagaaagga tccgtgcagt gccatcgtaa gccattttgg agcccggagc aaaaggaaat   1440
atcaccttgc ccaagcggtg cccctacagc tcatccgagt agggcccggg ggtcgaggca   1500
ttcggggccc agtgggggac gaggccagtc gaaggtctcg gaagtggagg ctccgccgag   1560
ctccggtcgg ggagtgcagg gatggccccc gggaccggag gtgagagctc gggaaagccg   1620
ctccgcccgg aacaaaggca tggggagagg gtgagcttgg gcgggagaga cccggcgggt   1680
accggtgccc tcgctgcctg ggtcgggctt ccacgcgcgc cccggaatgg aatacgctac   1740
tctgcagcct ccgaaactgc gagcgagtcc tgtaactccc ttgctctgtg attaattctc   1800
actaacaaga cttggcaaga tgtcgggcga atgatttttg gcttctgcac ggtccccacc   1860
gcgtgcgtgc acaaaccccc agccaaaagc cgcctctggg aaattaaatg caaaagagaa   1920
atggggatgg ggagggctgc tacgtgacca ggaaaaaggg atgcccagaa acatgaatcg   1980
gacccagagc tgctgaagtc ctttcaaaag gtcattcttt gcgggtacat tttccagggt   2040
ccagctccgc aacaaatgtg gaccctgtca tttcctgaaa ggataattca caactatgca   2100

agatagggtg aagacgtttc ccaaacccga aaaccttgtt tttcccccga ccagggttaa   2160
ataaacatct tttaggaagc gtggacagga gcgcagcctg ctctcctccc tcggaacacc   2220
attccggcaa ttaatgcctc cctttgggta gtaaagcaac aaacccccaca cctcactccg   2280
atcctgggct tcgggcggga ggactttctc tttcatcttc caagcagggg gttgcccacg   2340
ttcttgggga agctataaaa ctgatttaat ggctttagat gaaaatcgat cacgctaatg   2400
catacgctaa cgtctcagga atcgcatatt cagaaaggac tggccgggcc gaaagcgcac   2460
ggggagtctg gggctaggag gtgtcaggcc ccgctgggtg ggcagcagcg ctccggtccc   2520
ctctccactt gggtaaccgg gaaaaaccta cggggctgtc acgcggggaa gcgcgaaggt   2580
gccaagggat gaaagctcaa acccgagccc tggcctcctc agccggctat ttcctttggc   2640
gccgcccgcc tagcggcggg gtgcagcggc ggcacaggtg ccggtgtcgg gctggaggcg   2700
cggcgcaggc tgggcccgcg ggtagacggc gaaaggcgcc gcgcgctcca ttcacaaagt   2760
ccgggcgctg cccgccgctg gcggcgggtc ggaggccgcc tccctcttcc tctcggcctc   2820
ggttttatga atgggcctga tggcgagcac ccggcgccct gtttactccg ctctttgtga   2880
cgtcgagttc ccgtgaccgg gagccagcgg ccgcgctcca ttcaagctcc ggggaggggg   2940
tgggaggagg ggcccggagg gggcggggag tcagcgcggg gggcggggga cagcgcgggg   3000
ggcgggggac ggcgcggggc ccggaatgga acggggcggg gcctggcggg gtagtaccta   3060
gcgccccctc ccccgggagc gcggaggagc attaataaac ctctaagccg aggagaaaac   3120
tctggctggg gcagtgcgct gagcgccgga ggagcgtagg cagggcagcg ctggcgccag   3180
```

tggcgacagg agccgcgcga ccggcaaaaa tacacgggag gccgtcgccg aaaagagtcc 3240

gcggtcctct ctcgtaaaca cactctcctc caccggcgcc tccccctccg ctctgcgcgc 3300

cgccccggctg ggcgccccgag gccgctccga ctgctatgtg accgcgaggc tgcgggagga 3360

aggggacagg gaagaagagg ctctcccgcg ggagcccttg aggaccaagt ttgcggccac 3420

ttctgcaggc gtcccttctt agctctcgcc cgccccctttc tgcagcctag gcggcccggg 3480

ttctcttctc ttcctcgcgc gcccagccgc ctcggttccc ggcgaccatg gtgacgatgg 3540

aggagctgcg ggagatggac tgcagtgtgc tcaaaaggct gatgaaccgg gacgagaatg 3600

gcggcggcgc gggcggcagc ggcagccacg gcaccctggg gctgccgagc ggcggcaagt 3660

gcctgctgct ggactgcaga ccgttcctgg cgcacagcgc gggctacatc ctaggttcgg 3720

tcaacgtgcg ctgtaacacc atcgtgcggc ggcgggctaa gggctccgtg agcctggagc 3780

agatcctgcc cgccgaggag gaggtacgcg cccgcttgcg ctccggcctc tactcggcgg 3840

tcatcgtcta cgacgagcgc agcccgcgcg ccgagagcct ccgcgaggac agcaccgtgt 3900

cgctggtggt gcaggcgctg cgccgcaacg ccgagcgcac cgacatctgc ctgctcaaag 3960

gtaaacgagg gctccgggcg ctagctggag tcggggagat ggaggggtc ttggcggcct 4020

gcgccttatt ggcacgggaa ggagtccccg gcgtgattcg tggttgcggg gatccccgcg 4080

cgcccctttg tgtgtgttgg tgtgtagggg tttgcaaggt ttctgtgcaa gtgcgaggtt 4140

caagtcctca gggaaagtgc ggacgggttt ctccggtcac ctgcaattcc gcaccgtcct 4200

agcgaggccc cgtttattga cattaacggt cctagcctcg aggatgaagc gactcctctg 4260

cgcggtccat cttgagtccc gggaacagtt tccaagtacc acggggcgcg tggggacaag 4320

ataggcctgt tggtttggcg tgctggggcc ctacactgag ccggaccggg agaatcccag 4380

aggtggagaa aaggtgaaga ggggtgggcg ccgaggtaca attggccttg ccagtttcac 4440

tgaaaacaag accctggcaa atgcagaaag ggagattggg ttgcacacac tgtgtagagc 4500

gatctgtatc tgcgttcccg ctgcccaact ccaagctcag cttggaaggt ggccaatccc 4560

cgtcccgggc tagatccagg ctctgggagc gggaggctga aggactgggt atctggggga 4620

cgccgggcgg aaccggtcct ctagcgcctg caaatccggg cagcggagaa tgatcacctt 4680

ttgttcagcc cggattttcg actatctgat ggcggcgcgt gccggatttt agcgagccct 4740

ggcgaaactc tcggcggcga gactgtcttg tcacatttgg tagtcggaaa tcctggaaaa 4800

atcgcgtgct gtccgagtcc ctggagggac ctggcgcccg ccacacctgc agggcagggt 4860

gggtccgcgg ccctcgggct agggagcggg cagggcgcgt ggggtccggg cgccccccgc 4920

cgcgctgcgc ctccgcctcc cgccgccgca ttccgcgcat tcttccgcgc gggtagggtc 4980

tgcgcttccg agcccggtag gcagttcaga cccccccaca cccatcaaag agccgctcct 5040

cccccccgca ggcgccttcg ccgcctccct cccttccttt cctttccgct cctcttccga 5100

cctgtccacc cgggaggaag ggagctggaa agggggcgga aacctctccc ctccaaaaag 5160

cacaacaaaa ctgttcagtg cggaggagcc gggttcgccc ctgccggaca gcgggggct 5220

ttgttccccg cagttgtttc ctgcccattt gacctgtcag ctgctgggga aacgctgctg 5280

ttgacctttg gttgaactgc taaggcgatt ttgctgattt ttctttcttt ttccgcgagg 5340

gctgtctttt gctcctccaa atgagcccag tccccctccc ttctccccaa agcgctccaa 5400

gagaaagtgc caggaagggg cttgtcccgg aaggcctggc ggctgagcgg ggccaggtcc 5460

tggttaggcc accagggtgg gcgtccgcgc cattgtttga gcttgtcggc gctggtggga 5520

gagatgaggg caattcctct gggacgcaag tcccctcgaa tggccggggc tggccgggat 5580

gttccccgca cggcgctgcc ctcgagtccc cccgatggag agcgcgggcg cgccttcctt 5640

cgctggcgtc caaacccggg accagctaga acacagcagg gctgggactg ggttccagcc 5700

ccacgtggag tctggatttg ttttgttgtg ttttgctttc cttcctggaa gaaatcccga 5760

ggggaccgcc ctagagcggc agctccagga cctcggccct tgggcttccg ggggtgcagc 5820

cacttaggcc ccgctcccgg ggagagaggg attattttt aagatttatc cccagggcgc 5880

gcggcatttc cctgtccctc gtgaatcccg ttgagagtcc tccctcccca acctcctcca 5940

tttccccagc cagaccgatt cgagagccct ggagattctg ggcgaggcta gtgactgggt 6000

agtacaggcc tctaggtaaa atgacagccc tctggtgggt ggcgaggtgc ctggccaaca 6060

taagagaagt actttaaggg ctgtttcctc ctggagcctg cactgcctct cgtagtcggc 6120

cccgacgcgg gcagggagaa tcatcttctg catctcaaag tggacatcct tgcagatgga 6180

ggtgtgcagg cctgggccac agatttagct tcggtctctt ctagaatagc ccctcctagc 6240

cagaagttca gcttctgctt ccaaagggag cctgcccttg gaagcaagca cctaaaattg 6300

agaggtgggg gaatcttcca gcctgctctg ttgtggtgaa catcgttgag gttaactggg 6360

ccctattcgg tttagcttag ctgattttga catatgtgtt cagcagaggc catctttaac 6420

ccagttcctc ttcttcctct ttgccctcta atggaaaggc atccctagag aggtcagaag 6480

gaaaaagtca gtatctgctc ccacagctct gcgggaaggg gctggggaag gaaatcaagg 6540

tgtctcccag gtctccagat acacctccct tccatgcctg gaagccagtg cagtggagca 6600

aatgtggttt cccgggaggg ggctctgggg cagagaccta aaggggaatt ttgagaagca 6660

aaggaagaaa aagcccctta gattatcttt agtaaatgcc tgaattggtg aagggttgga 6720

gaatttgttt cctactggag taattcattt tattttccag cgagggaatg tatgtatgtc 6780

tcatttcagg tgtgcctgtg cgtgtctgta aagcaggaca ggaagccagt ggaaggcttg 6840

atggttggtg tctgccattc ttggaggggt taggagaagc agtaaaagca acaagagtat 6900

tttcagaagt gatagtagca tgtccagcca aagctatcta attctattaa ttgcatttta 6960

aaattcagtt tttctatttc tgccacgttg aagggttact tcaaccacgt tgaactggtt 7020

actccatgca ggtggcagga ggtaaacttc gcttaatatc aagaaagtac agcctagcct 7080

ttacatttcc tcagattacc cgacttttgc taaaagcaat aacgaaaaat ctaatgtttt 7140

ctatctttgc tagtaaagga tttccagtgg ggtaattttt ttaaaactca tttaatttaa 7200

aatcttgtaa ggcattttta gagtcagttt ggaaatttta gtttttcaga ttttatcaca 7260

atacacttct tgaaatccct ttctctctca ttcgtaaatc cttggattag agtttgagac 7320

tcccctggtc attagagaaa gacaaaaaga gctttttctt accctattct ttgcctttat 7380

taaccccaaa taacaaaata atctgttatg caaaaagatt gcaggtataa aaacattacc 7440

ttcctttcca cctacctaaa acttccctca gcctttgtca gttctaattt aaaagctgtc 7500

ctggagctgt tggagaaatg gggaagatca tctagatatt ccagtttctc cttccactca 7560

ccctatagta ccggatagca tccaatagcc cagggcctga ccatacagcc catgatatta 7620

ccctttctgt agaaaacagt cactaaggcc actgatgaat gtggggggag aggaacacaa 7680

ttaacagctc tggaaactct ggtgacattt ttctgttttt tctctctcta gccccaccat 7740

tgctctctct gtcttcagtt ccccaggagg gcaatggcat caaacagcac agctctgggg 7800

gatgtcaata ttgcatacct tttctaccta aagggaaaat gactcgcttt tctgcttgca 7860

aatatggtag tttctgctta caaatgtaat acaatgccca tgacagccaa ggactggaag 7920

cataagttgc taggtcttac aggtgatttt ttacaatgaa gcaaactcac tatgttagac 7980

accatttaca ttggatgtct ccaactaaca aaagtaacta aagacagatg taggtgtaaa 8040

ttgagagtga aatttgaccc tttagaccgt cacaacttcc ttgggcttat cctgggtgct 8100

tataggagag gtgggctcca cccacaaaaa tggactgctc agaaaaatga gggagagaga 8160

aagggtggcc actttcccga gccaagaaat tccttgaaaa aaaatcagaa catctgaaac 8220

cagagagccg atttccttac cgggaggcag ttcctggcta acgaagagga agcacgatgg 8280

gaagaaaagt tcactccaac ggaagccagt ttgctgaaca tagcagatcg cccaggagga 8340

ctgggagaga ctgcaaacca gttcgagccc ccagcatggc gttaggtgtc agccagctgg 8400

caggaaggtc caggtgtctg tgttcagagt ctcaaggtga gaagttcact tagttggatt 8460

ttaaatacag ctcagcattt acataggtgc ttactaagtc aacagtcaca taaagtagag 8520

ttcccaaagg caaatggcct tcaaacaacc ccagactttc ttgcaagagc atgacttttc 8580

tggcaagagc aaggccatga gccctgcctg ggacctggca ctattctgag tgttggggc 8640

ataatgggga aaaagtggca gccacattaa gattcatgac caaggccggg tgtggtggct 8700

cacacctgca atcctagcac tttgagagac tgaggcaggc agatcacttg aggccaggac 8760

aacatggcaa acttggtctc tacaaaaaat gcaaaaatta gccgggcgag atggcatgtg 8820

cctgtaatct cagctactca ggaggctgaa gcaaggagat tgcttgagcc cagggaagtc 8880

aaggctacag tgagccataa tcgtgccact gcactgcagc ctgggtgaga gagtgacagt 8940

gacagaccct gtctggggaa aaaaaaaaaa aaaagatcca tgaccatagt caccaccaaa 9000

ggacagtctt ccccatcaca aatcccagta acagtcctat tatggtgata 9050

<210> 2
<211> 559
<212> DNA
<213> Artificial

<220>
<223> CG-rich DUSP4 segment

<400> 2
cgctttcgca attcaagtgg ctctcggctt aaacacatta agtcccaagc tgtgactccc 60

ttccagtgaa cagccgcgta ggcgcgggca cgcacacgtg gacacacaca ctcacacgcg 120

ccctcccaca cccctttccg tgtgaatcgc ctgcgggatt tagggcttct ccaccagcga 180

ttactgtcag gtgcagaaag caagcctctt tgttctatgg gcattttcct cccccctggat 240

ttgactgtta tttactggcc acgtgagcgc caggcgcggg tccggcgggg aaggcgaccc 300

cagaacaggg tagacaaaag ctggcccaga gcgcgacccg tgcgccccag ggacgtctcc 360

ccagctcctg cgacacgcgt gaaattggcc cggacctgcc tcctgagagg cttttcccag 420

aaatgtcttg ttaaagcctt tgtaaaggaa tcagtgttta cttgcgccgg gagagctccg 480

gccctgggtc ctggcaggcg gcggatttct gagctgctgg agagcaaccc gggaaagcaa 540

aatcagcctt ggactgtcg 559

<210> 3
<211> 6100
<212> DNA
<213> Artificial

<220>
<223> unmodified reverse complement of DUSP4

<400> 3
ggctaggacc gttaatgtca ataaacgggg cctcgctagg acggtgcgga attgcaggtg 60

accggagaaa cccgtccgca ctttccctga ggacttgaac ctcgcacttg cacagaaacc 120

ttgcaaaccc ctacacacca acacacacaa aggggcgcgc ggggatcccc gcaaccacga 180

atcacgccgg ggactccttc ccgtgccaat aaggcgcagg ccgccaagac cccctccatc 240

tccccgactc cagctagcgc ccggagccct cgtttacctt tgagcaggca gatgtcggtg 300

cgctcggcgt tgcggcgcag cgcctgcacc accagcgaca cggtgctgtc ctcgcggagg 360

ctctcggcgc gcgggctgcg ctcgtcgtag acgatgaccg ccgagtagag gccggagcgc 420

```
aagcgggcgc gtacctcctc ctcggcgggc aggatctgct ccaggctcac ggagccctta     480
gcccgccgcc gcacgatggt gttacagcgc acgttgaccg aacctaggat gtagcccgcg     540
ctgtgcgcca ggaacggtct gcagtccagc agcaggcact tgccgccgct cggcagcccc     600
agggtgccgt ggctgccgct gccgcccgcg ccgccgccat tctcgtcccg gttcatcagc     660
cttttgagca cactgcagtc catctcccgc agctcctcca tcgtcaccat ggtcgccggg     720
aaccgaggcg gctgggcgcg cgaggaagag aagagaaccc gggccgccta ggctgcagaa     780
aggggcgggc gagagctaag aagggacgcc tgcagaagtg gccgcaaact tggtcctcaa     840
gggctcccgc gggagagcct cttcttccct gtccccttcc tcccgcagcc tcgcggtcac     900
atagcagtcg gagcggcctc gggcgcccag ccgggcggcg cgcagagcgg agggggaggc     960
gccggtggag gagagtgtgt ttacgagaga ggaccgcgga ctcttttcgg cgacggcctc    1020
ccgtgtattt ttgccggtcg cgcggctcct gtcgccactg gcgccagcgc tgccctgcct    1080
acgctcctcc ggcgctcagc gcactgcccc agccagagtt ttctcctcgg cttagaggtt    1140
tattaatgct cctccgcgct cccgggggag gggcgctag gtactacccc gccaggcccc     1200
gccccgttcc attccgggcc ccgcgccgtc ccccgccccc cgcgctgtcc cccgcccccc    1260
gcgctgactc cccgccccct ccgggccccct cctcccaccc cctccccgga gcttgaatgg   1320
agcgcggccg ctggctcccg gtcacgggaa ctcgacgtca caaagagcgg agtaaacagg    1380
gcgccgggtg ctcgccatca ggcccattca taaaaccgag gccgagagga agagggaggc    1440
ggcctccgac ccgccgccag cggcgggcag cgcccggact ttgtgaatgg agcgcgcggc    1500
gcctttcgcc gtctacccgc gggcccagcc tgcgccgcgc ctccagcccg acaccggcac    1560
ctgtgccgcc gctgcacccc gccgctaggc gggcggcgcc aaaggaaata gccggctgag    1620
gaggccaggg ctcgggtttg agctttcatc ccttggcacc ttcgcgcttc cccgcgtgac    1680
agccccgtag gtttttcccg gttacccaag tggagagggg accggagcgc tgctgcccac    1740
ccagcggggc ctgacacctc ctagccccag actccccgtg cgctttcggc ccggccagtc    1800
ctttctgaat atgcgattcc tgagacgtta gcgtatgcat tagcgtgatc gattttcatc    1860
taaagccatt aaatcagttt tatagcttcc ccaagaacgt gggcaacccc ctgcttggaa    1920
gatgaaagag aaagtcctcc cgcccgaagc ccaggatcgg agtgaggtgt ggggtttgtt    1980
gctttactac ccaaagggag gcattaattg ccggaatggt gttccgaggg aggagagcag    2040
gctgcgctcc tgtccacgct tcctaaaaga tgtttattta accctggtcg ggggaaaaac    2100
aaggttttcg ggtttgggaa acgtcttcac cctatcttgc atagttgtga attatccttt    2160

caggaaatga cagggtccac atttgttgcg gagctggacc ctgcaaaatg tacccgcaaa    2220
gaatgacctt ttgaaaggac ttcagcagct ctgggtccga ttcatgtttc tgggcatccc    2280
```

tttttcctgg tcacgtagca gccctcccca tccccatttc tcttttgcat ttaatttccc 2340
agaggcggct tttggctggg ggtttgtgca cgcacgcggt ggggaccgtg cagaagccaa 2400
aaatcattcg cccgacatct tgccaagtct tgttagtgag aattaatcac agagcaaggg 2460
agttacagga ctcgctcgca gtttcggagg ctgcagagta gcgtattcca ttccggggcg 2520
cgcgtggaag cccgacccag gcagcgaggg caccggtacc cgccgggtct ctcccgccca 2580
agctcaccct ctccccatgc ctttgttccg ggcggagcgg ctttcccgag ctctcacctc 2640
cggtcccggg ggccatccct gcactccccg accggagctc ggcggagcct ccacttccga 2700
gaccttcgac tggcctcgtc ccccactggg ccccgaatgc ctcgaccccc gggccctact 2760
cggatgagct gtaggggcac cgcttgggca aggtgatatt tccttttgct ccgggctcca 2820
aaatggctta cgatggcact gcacggatcc tttctctttt taaaatttag tattttattc 2880
atcatggatt ttttgcattt ttttttcttt taaagaggca ttgcatcaag atatttcttt 2940
tgattactga cttttcggcg cccttcttaa atgttgcgct caaggcttgt gctcacccta 3000
gtccaggccc tggaaggagc cacaactgtg gactgaccga ggctgcccgg tggcaggacc 3060
ctgggcgtcg ggcactccgg gtgggagatc agtggtcaaa gccgcacggc gtggcctccc 3120
tgtggggagg gcaaaaggtg tctgagtcca gtttcccatt ctgctgaatg ggactgtggg 3180
ggtctatcta ataactcgct ttcgataagg agcaaggagc cccaggggcc ccgacagtcc 3240
aaggctgatt ttgctttccc gggttgctct ccagcagctc agaaatccgc cgcctgccag 3300
gacccagggc cggagctctc ccggcgcaag taaacactga ttcctttaca aaggctttaa 3360
caagacattt ctgggaaaag cctctcagga ggcaggtccg ggccaatttc acgcgtgtcg 3420
caggagctgg ggagacgtcc ctggggcgca cgggtcgcgc tctgggccag cttttgtcta 3480
ccctgttctg ggtcgcctt ccccgccgga cccgcgcctg gcgctcacgt ggccagtaaa 3540
taacagtcaa atccaggggg aggaaaatgc ccatagaaca aagaggcttg ctttctgcac 3600
ctgacagtaa tcgctggtgg agaagcccta aatcccgcag gcgattcaca cggaaagggg 3660
tgtgggaggg cgcgtgtgag tgtgtgtgtc cacgtgtgcg tgcccgcgcc tacgcggctg 3720
ttcactggaa gggagtcaca gcttgggact taatgtgttt aagccgagag ccacttgaat 3780
tgcgaaagcg aaggcctgat ttttaaaaaa gactaatttt taaaatccac accccattgc 3840
caactcagga attggccttc agttgtgtga atttcagcaa aggagattta agttaccata 3900
ttgtatttat cgggatactg tgtaaacacc ctagatatat attttttaat tgtatgtttg 3960
tgaaccattg gtccacagtt aaatgagtac aacgcgaggg ctactccatg ccggctatag 4020
ataaaacctg tctccaattc gtctgcagtc tgtggacgaa acataaccat tttaaacagg 4080
aaccttgtga aacagcttgt caatgagcag atcttggaga aaacaaaggc aatttggagc 4140
aagacacgga atgtcatttc aaagttaatt taattaacta aggcatattt gtaggaattg 4200

```
ggatgggatt gcccaaggag aggtctttta tttatattca gcagtaaacc tcggggtgaa   4260
agaactttta aaaattcgct gtgacaagta aatccagctt ctctttcatc agtcagtcct   4320
tagaccgacc ttattagtca ctgtcaggct ccagcaaaac acctgctctt gtttaggaac   4380
atttctttcc aattaggaag ttgtatgatc ggggaatcta aagcccggag tagaatcccc   4440
agagagaagt acagcttcta ttctagtttt gatgccaacc tctgactcac tgctctgtcc   4500
caaagtgcct gaataaaact tgcctgaaaa atcattaatg aattaattaa ggtggtgatg   4560
gaaagaaaag tcctgtggct tttcctttct tgagcaaaga ggcagccttg ctattacgtc   4620
gaactggaac aggaataatc cattttgcac agatcttgtt ggttaggctt cttagatcaa   4680
ctaattaggg aacttgcccc ctggtagtgg gaaggctcaa aagaactggg agaccctctc   4740
ctgctcctga aagcagagaa gcctgtggtc tacacagcgt gttcttaggc aacgtttaga   4800
aatggaattg agaaatgcct gtccttccct acgtacctct gcccatcaag tcaggactga   4860
aagcgaagga aagtcttcaa agttcacact gaacctaacc aaaaactctc aaagacatag   4920
cctgaggatt tctttttttta agtcctgact ttgttacaga tctttttttgt tttctatgct   4980
tgatactcta gcacttatta atgatttaaa aggctatgta tcggccaggc gaggtggctc   5040
acgctagtaa tcccagcact ttggaagacc gaagtgggca gatcacctga ggtcaggagt   5100
ttaagaccag cctggtcaac atggtgaaac cctgtctctt ctaaaaatac aaaattagcc   5160
agcatgacgg gcgcgcaccc ataatcccag ctacactgga ggctgaggca ggagaatcgc   5220
ttgaacccgg gaggtagagg ttgcagtgag ctgagatccc ctcactgcac tccagcctgg   5280
gcgacaagag caaaactcca tctcaaaata aataaataaa tacaaataaa aaataataaa   5340
agattaagtt tctaacccag ggtctctgca gaaaggtttt aatgcattgc tttatttact   5400
gctttatttt aaaactattc aatctaggct atgccaccat ctagcttggt ggccttgggc   5460
aagacagtct cctttggcct cactttcttc acctgcaaaa tgaagagttt gggcaggatg   5520
gtttctcagg tctggtctat ttccgaccct gtatgattct gagcagaaga agacttctca   5580
gctccgagtc caagtttcat aggtttccca tctcgccctc tgaagagcca agcacactat   5640
aatgccataa gataaaggac gatctctagc tcctccttgt ccctgcttta gaatataaac   5700
tcatggacag cagagctcat agtttttatt tatttgttta gtcacgtgct aacgtcccaa   5760
gcattttgat aaaacgtctt caaaatttca gaaacaaatt ttggaaagaa acacttctcc   5820
aaacgggata aaaatgaact cggaggctgg gcatggatgg tggctcatgc ctgtaatccc   5880
ccagcacttt gggaggctgg cggatcactt gaggtcagga gttggagacc agcctgacca   5940
acattaaaaa tacaaaaatt agtcaggcat gatggtgggt gcctgtagtc ccagctactc   6000
gtgaggctga ggcaaaagaa tcccttgacc cggagaggca gaggttgcag tgagtcaaga   6060
```

ttgggccact gcactcctgc ctggacaggg cgagacagcg 6100

<210> 4
<211> 6100
<212> DNA
<213> Artificial

<220>
<223> modified reverse complement of DUSP4 (unmethylated)

<400> 4
ggttaggatt gttaatgtta ataaatgggg ttttgttagg atggtgtgga attgtaggtg 60
attggagaaa tttgtttgta ttttttttga ggatttgaat tttgtatttg tatagaaatt 120
ttgtaaattt ttatatatta atatatataa aggggtgtgt ggggattttt gtaattatga 180
attatgttgg ggattttttt ttgtgttaat aaggtgtagg ttgttaagat tttttttatt 240
tttttgattt tagttagtgt ttggagtttt tgtttatttt tgagtaggta gatgttggtg 300
tgtttggtgt tgtggtgtag tgtttgtatt attagtgata tggtgttgtt tttgtggagg 360
tttttggtgt gtgggttgtg tttgttgtag atgatgattg ttgagtagag gttggagtgt 420
aagtgggtgt gtattttttt tttggtgggt aggatttgtt ttaggtttat ggagttttta 480
gtttgttgtt gtatgatggt gttatagtgt atgttgattg aatttaggat gtagtttgtg 540
ttgtgtgtta ggaatggttt gtagtttagt agtaggtatt tgttgttgtt tggtagtttt 600
agggtgttgt ggttgttgtt gttgtttgtg ttgttgttat ttttgttttg gtttattagt 660
tttttgagta tattgtagtt tatttttttgt agttttttta ttgttattat ggttgttggg 720
aattgaggtg gttgggtgtg tgaggaagag aagagaattt gggttgttta ggttgtagaa 780
aggggtgggt gagagttaag aagggatgtt tgtagaagtg gttgtaaatt tggtttttaa 840
gggtttttgt gggagagttt tttttttttt gttttttttt ttttgtagtt ttgtggttat 900
atagtagttg gagtggtttt gggtgtttag ttgggtggtg tgtagagtgg aggggggaggt 960
gttggtggag gagagtgtgt ttatgagaga ggattgtgga tttttttttgg tgatggtttt 1020
ttgtgtattt ttgttggttg tgtggttttt gttgttattg gtgttagtgt tgttttgttt 1080
atgttttttt ggtgtttagt gtattgtttt agttagagtt tttttttttgg tttagaggtt 1140
tattaatgtt tttttgtgtt tttgggggag ggggtgttag gtattatttt gttaggtttt 1200
gttttgtttt attttgggtt ttgtgttgtt ttttgttttt tgtgttgttt tttgtttttt 1260
gtgttgattt tttgtttttt ttgggttttt tttttatttt ttttttttgga gtttgaatgg 1320
agtgtggttg ttggtttttg gttatgggaa tttgatgtta taaagagtgg agtaaatagg 1380
gtgttgggtg tttgttatta ggtttattta taaaattgag gttgagagga agagggaggt 1440
ggtttttgat ttgttgttag tggtgggtag tgtttggatt ttgtgaatgg agtgtgtggt 1500

gtttttttgtt gtttatttgt gggtttagtt tgtgttgtgt ttttagtttg atattggtat 1560
ttgtgttgtt gttgtatttt gttgttaggt gggtggtgtt aaaggaaata gttggttgag 1620
gaggttaggg tttgggtttg agtttttatt ttttggtatt tttgtgtttt tttgtgtgat 1680
agttttgtag gttttttttg gttatttaag tggagagggg attggagtgt tgttgtttat 1740
ttagtggggt ttgatatttt ttagttttag atttttttgtg tgtttttggt ttggttagtt 1800
tttttttgaat atgtgatttt tgagatgtta gtgtatgtat tagtgtgatt gatttttatt 1860
taaagttatt aaattagttt tatagttttt ttaagaatgt gggtaatttt ttgtttggaa 1920
gatgaaagag aaagtttttt tgtttgaagt ttaggattgg agtgaggtgt ggggtttgtt 1980
gttttattat ttaaagggag gtattaattg ttggaatggt gttttgaggg aggagagtag 2040
gttgtgtttt tgtttatgtt ttttaaaaga tgtttattta attttggttg ggggaaaaat 2100
aaggtttttg ggtttgggaa atgtttttat tttattttgt atagttgtga attatttttt 2160
taggaaatga tagggtttat atttgttgtg gagttggatt ttggaaaatg tatttgtaaa 2220
gaatgatttt ttgaaaggat tttagtagtt ttgggtttga tttatgtttt tgggtatttt 2280
tttttttttgg ttatgtagta gttttttttta tttttatttt ttttttgtat ttaatttttt 2340
agaggtggtt tttggttggg ggtttgtgta tgtatgtggt ggggattgtg tagaagttaa 2400
aaattatttg tttgatattt tgttaagttt tgttagtgag aattaattat agagtaaggg 2460
agttatagga tttgtttgta gttttggagg ttgtagagta gtgtatttta ttttggggtg 2520
tgtgtggaag tttgatttag gtagtgaggg tattggtatt tgttgggttt tttttgttta 2580
agtttatttt ttttttatgt ttttgttttg ggtggagtgg ttttttttgag tttttatttt 2640
tggttttggg ggttattttt gtattttttg attggagttt ggtggagttt ttattttttga 2700
gatttttgat tggttttgtt ttttattggg ttttgaatgt tttgattttt gggttttatt 2760
tggatgagtt gtaggggtat tgtttgggta aggtgatatt ttttttttgtt ttgggtttta 2820
aaatggttta tgatggtatt gtatggattt tttttttttt taaaatttag tattttattt 2880
attatggatt ttttgtattt tttttttttt taaagaggta ttgtattaag atattttttt 2940
tgattattga tttttttggtg ttttttttaa atgttgtgtt taaggtttgt gtttatttta 3000
gtttaggttt tggaaggagt tataattgtg gattgattga ggttgtttgg tggtaggatt 3060
ttgggtgttg ggtattttgg gtgggagatt agtggttaaa gttgtatggt gtggtttttt 3120
tgtggggagg gtaaaaggtg tttgagttta gtttttttatt ttgttgaatg ggattgtggg 3180
ggtttattta ataatttgtt tttgataagg agtaaggagt tttaggggtt ttgatagttt 3240
aaggttgatt ttgttttttt gggttgtttt ttagtagttt agaaatttgt tgtttgttag 3300
gatttagggt tggagttttt ttggtgtaag taaatattga ttttttttata aaggttttaa 3360
taagatattt ttgggaaaag ttttttagga ggtaggtttg ggttaatttt atgtgtgttg 3420

taggagttgg ggagatgttt ttggggtgta tgggttgtgt tttgggttag tttttgttta 3480
ttttgttttg gggttgtttt ttttgttgga tttgtgtttg gtgtttatgt ggttagtaaa 3540
taatagttaa atttaggggg aggaaaatgt ttatagaata aagaggtttg ttttttgtat 3600
ttgatagtaa ttgttggtgg agaagtttta aattttgtag gtgatttata tggaaagggg 3660
tgtgggaggg tgtgtgtgag tgtgtgtgtt tatgtgtgtg tgtttgtgtt tatgtggttg 3720
tttattggaa gggagttata gtttgggatt taatgtgttt aagttgagag ttatttgaat 3780
tgtgaaagtg aaggtttgat ttttaaaaaa gattaatttt taaaatttat attttattgt 3840
taatttagga attggttttt agttgtgtga attttagtaa aggagattta agttattata 3900
ttgtatttat tgggatattg tgtaaatatt ttagatatat atttttaat tgtatgtttg 3960
tgaattattg gtttatagtt aaatgagtat aatgtgaggg ttattttatg ttggttatag 4020
ataaaatttg tttttaattt gtttgtagtt tgtggatgaa atataattat tttaaatagg 4080
aattttgtga aatagtttgt taatgagtag attttggaga aaataaaggt aatttggagt 4140
aagatatgga atgttatttt aaagttaatt taattaatta aggtatattt gtaggaattg 4200
ggatggattt gtttaaggag aggtttttta tttatattta gtagtaaatt ttggggtgaa 4260
agaattttta aaaatttgtt gtgataagta aatttagttt tttttttatt agttagtttt 4320
tagattgatt ttattagtta ttgttaggtt ttagtaaaat atttgttttt gtttaggaat 4380
attttttttt aattaggaag ttgtatgatt ggggaattta aagtttggag tagaattttt 4440
agagagaagt atagttttta ttttagtttt gatgttaatt tttgatttat tgttttgttt 4500
taaagtgttt gaataaaatt tgtttgaaaa attattaatg aattaattaa ggtggtgatg 4560
gaaagaaaag ttttgtggtt tttttttttt tgagtaaaga ggtagttttg ttattatgtt 4620
gaattggaat aggaataatt tattttgtat agattttgtt ggttaggttt tttagattaa 4680
ttaattaggg aatttgtttt ttggtagtgg gaaggtttaa aagaattggg agattttttt 4740
ttgtttttga aagtagagaa gtttgtggtt tatatagtgt gtttttaggt aatgtttaga 4800
aatggaattg agaaatgttt gttttttttt atgtattttt gtttattaag ttaggattga 4860
aagtgaagga aagtttttaa agtttatatt gaatttaatt aaaaattttt aaagatatag 4920
tttgaggatt ttttttttta agttttgatt ttgttataga ttttttttgt tttttatgtt 4980
tgatatttta gtatttatta atgatttaaa aggttatgta ttggttaggt gaggtggttt 5040
atgttagtaa ttttagtatt ttggaagatt gaagtgggta gattatttga ggttaggagt 5100
ttaagattag tttggttaat atggtgaaat tttgtttttt ttaaaaatat aaaattagtt 5160
agtatgatgg gtgtgtattt ataattttag ttatattgga ggttgaggta ggagaattgt 5220
ttgaatttgg gaggtagagg ttgtagtgag ttgagatttt tttattgtat tttagtttgg 5280

gtgataagag taaaatttta ttttaaaata aataaataaa tataaataaa aaataataaa 5340

agattaagtt tttaatttag ggtttttgta gaaaggtttt aatgtattgt tttatttatt 5400

gttttatttt aaaattattt aatttaggtt atgttattat ttagtttggt ggttttgggt 5460

aagatagttt tttttggttt tatttttttt atttgtaaaa tgaagagttt gggtaggatg 5520

gtttttttagg tttggtttat ttttgatttt gtatgatttt gagtagaaga agattttttta 5580

gttttgagtt taagttttat aggtttttta ttttgttttt tgaagagtta agtatattat 5640

aatgttataa gataaaggat gatttttagt tttttttttgt ttttgtttta gaatataaat 5700

ttatggatag tagagtttat agtttttatt tatttgttta gttatgtgtt aatgttttaa 5760

gtattttgat aaaatgtttt taaaatttta gaaataaatt ttggaaagaa atattttttt 5820

aaatgggata aaaatgaatt tggaggttgg gtatggatgg tggtttatgt ttgtaatttt 5880

ttagtatttt gggaggttgg tggattattt gaggttagga gttggagatt agtttgatta 5940

atattaaaaa tataaaaatt agttaggtat gatggtgggt gtttgtagtt ttagttattt 6000

gtgaggttga ggtaaaagaa tttttgatt tggagaggta gaggttgtag tgagttaaga 6060

ttgggttatt gtatttttgt ttggataggg tgagatagtg 6100

<210> 5
<211> 6100
<212> DNA
<213> Artificial

<220>
<223> modified reverse complement of DUSP4 (methylated)

<400> 5
ggttaggatc gttaatgtta ataaacgggg tttcgttagg acggtgcgga attgtaggtg 60

atcggagaaa ttcgttcgta ttttttttga ggatttgaat ttcgtatttg tatagaaatt 120

ttgtaaattt ttatatatta atatatataa aggggcgcgc ggggattttc gtaattacga 180

attacgtcgg ggattttttt tcgtgttaat aaggcgtagg tcgttaagat ttttttatt 240

ttttcgattt tagttagcgt tcggagtttt cgtttatttt tgagtaggta gatgtcggtg 300

cgttcggcgt tgcggcgtag cgtttgtatt attagcgata cggtgttgtt ttcgcggagg 360

ttttcggcgc gcgggttgcg ttcgtcgtag acgatgatcg tcgagtagag gtcggagcgt 420

aagcgggcgc gtattttttt ttcggcgggt aggatttgtt ttaggtttac ggagtttta 480

gttcgtcgtc gtacgatggt gttatagcgt acgttgatcg aatttaggat gtagttcgcg 540

ttgtgcgtta ggaacggttt gtagtttagt agtaggtatt tgtcgtcgtt cggtagtttt 600

agggtgtcgt ggttgtcgtt gtcgttcgcg tcgtcgttat tttcgtttcg gtttattagt 660

tttttgagta tattgtagtt tatttttcgt agtttttta tcgttattat ggtcgtcggg 720

aatcgaggcg gttgggcgcg cgaggaagag aagagaattc gggtcgttta ggttgtagaa 780

aggggcgggc gagagttaag aagggacgtt tgtagaagtg gtcgtaaatt tggtttttaa 840
gggttttcgc gggagagttt tttttttttt gttttttttt tttcgtagtt tcgcggttat 900
atagtagtcg gagcggtttc gggcgtttag tcgggcggcg cgtagagcgg agggggaggc 960
gtcggtggag gagagtgtgt ttacgagaga ggatcgcgga tttttttcgg cgacggtttt 1020
tcgtgtattt ttgtcggtcg cgcggttttt gtcgttattg gcgttagcgt tgttttgttt 1080
acgtttttc ggcgtttagc gtattgtttt agttagagtt tttttttcgg tttagaggtt 1140
tattaatgtt ttttcgcgtt ttcgggggag ggggcgttag gtattatttc gttaggtttc 1200
gtttcgtttt atttcgggtt tcgcgtcgtt tttcgttttt cgcgttgttt ttcgtttttc 1260
gcgttgattt ttcgtttttt tcgggttttt tttttttattt tttttcgga gtttgaatgg 1320
agcgcggtcg ttggttttcg gttacgggaa ttcgacgtta taaagagcgg agtaaatagg 1380
gcgtcgggtg ttcgttatta ggtttattta taaaatcgag gtcgagagga agagggaggc 1440
ggttttcgat tcgtcgttag cggcgggtag cgttcggatt ttgtgaatgg agcgcgcggc 1500
gtttttcgtc gtttattcgc gggtttagtt tgcgtcgcgt ttttagttcg atatcggtat 1560
ttgtgtcgtc gttgtatttc gtcgttaggc gggcggcgtt aaaggaaata gtcggttgag 1620
gaggttaggg ttcgggtttg agttttattt ttttggtatt ttcgcgtttt ttcgcgtgat 1680
agtttcgtag gtttttttcg gttatttaag tggagagggg atcggagcgt tgttgtttat 1740
ttagcggggt ttgatatttt ttagttttag atttttcgtg cgttttcggt tcggttagtt 1800
tttttgaat atgcgatttt tgagacgtta gcgtatgtat tagcgtgatc gatttttatt 1860
taaagttatt aaattagttt tatagttttt ttaagaacgt gggtaatttt ttgtttggaa 1920
gatgaaagag aaagtttttt cgttcgaagt ttaggatcgg agtgaggtgt ggggtttgtt 1980
gttttattat ttaaagggag gtattaattg tcggaatggt gtttcgaggg aggagagtag 2040
gttgcgtttt tgtttacgtt ttttaaaaga tgtttattta attttggtcg ggggaaaaat 2100
aaggttttcg ggtttgggaa acgtttttat tttattttgt atagttgtga attatttttt 2160
taggaaatga tagggtttat atttgttgcg gagttggatt ttggaaaatg tattcgtaaa 2220
gaatgatttt ttgaaaggat tttagtagtt ttgggttcga tttatgtttt tgggtatttt 2280
tttttttgg ttacgtagta gtttttttta tttttatttt tttttgtat ttaattttt 2340
agaggcggtt tttggttggg ggtttgtgta cgtacgcggt ggggatcgtg tagaagttaa 2400
aaattattcg ttcgatattt tgttaagttt tgttagtgag aattaattat agagtaaggg 2460
agttatagga ttcgttcgta gtttcggagg ttgtagagta gcgtatttta tttcggggcg 2520
cgcgtggaag ttcgatttag gtagcgaggg tatcggtatt cgtcgggttt ttttcgttta 2580
agtttatttt ttttttatgt ttttgtttcg ggcggagcgg ttttttcgag tttttatttt 2640

cggtttcggg ggttattttt gtatttttcg atcggagttc ggcggagttt ttatttttcga 2700
gattttcgat tggtttcgtt ttttattggg tttcgaatgt ttcgattttc gggtttttatt 2760
cggatgagtt gtaggggtat cgtttgggta aggtgatatt tttttttgtt tcgggtttta 2820
aaatggttta cgatggtatt gtacggattt ttttttttttt taaaatttag tatttttattt 2880
attatggatt ttttgtattt tttttttttt taaagaggta ttgtattaag atattttttt 2940
tgattattga tttttcggcg ttttttttaa atgttgcgtt taaggtttgt gtttatttta 3000
gtttaggttt tggaaggagt tataattgtg gattgatcga ggttgttcgg tggtaggatt 3060
ttgggcgtcg ggtatttcgg gtgggagatt agtggttaaa gtcgtacggc gtggtttttt 3120
tgtggggagg gtaaaaggtg tttgagttta gtttttttatt ttgttgaatg ggattgtggg 3180
ggtttattta ataattcgtt ttcgataagg agtaaggagt tttaggggtt tcgatagttt 3240
aaggttgatt ttgttttttc gggttgtttt ttagtagttt agaaattcgt cgtttgttag 3300
gatttagggt cggagttttt tcggcgtaag taaatattga tttttttata aaggttttaa 3360
taagatattt ttgggaaaag tttttttagga ggtaggttcg ggttaatttt acgcgtgtcg 3420
taggagttgg ggagacgttt ttggggcgta cgggtcgcgt tttgggttag tttttgttta 3480
ttttgttttg gggtcgtttt tttcgtcgga ttcgcgtttg gcgtttacgt ggttagtaaa 3540
taatagttaa atttaggggg aggaaaatgt ttatagaata aagaggtttg tttttttgtat 3600
ttgatagtaa tcgttggtgg agaagtttta aatttcgtag gcgatttata cggaaagggg 3660
tgtgggaggg cgcgtgtgag tgtgtgtgtt tacgtgtgcg tgttcgcgtt tacgcggttg 3720
tttattggaa gggagttata gtttgggatt taatgtgttt aagtcgagag ttatttgaat 3780
tgcgaaagcg aaggtttgat ttttaaaaaa gattaatttt taaaatttat attttattgt 3840
taatttagga attggttttt agttgtgtga atttagtaa aggagattta agttattata 3900
ttgtatttat cgggatattg tgtaaatatt ttagatatat atttttttaat tgtatgtttg 3960
tgaattattg gtttatagtt aaatgagtat aacgcgaggg ttattttatg tcggttatag 4020
ataaaatttg tttttaattc gtttgtagtt tgtggacgaa atataattat tttaaatagg 4080
aattttgtga aatagtttgt taatgagtag attttggaga aaataaaggt aatttggagt 4140
aagatacgga atgttatttt aaagttaatt taattaatta aggtatattt gtaggaattg 4200
ggatgggatt gtttaaggag aggtttttta tttatattta gtagtaaatt tcggggtgaa 4260
agaattttta aaaattcgtt gtgataagta aatttagttt tttttttatt agttagtttt 4320
tagatcgatt ttattagtta ttgttaggtt ttagtaaaat atttgttttt gtttaggaat 4380
atttttttt aattaggaag ttgtatgatc ggggaattta aagttcggag tagaattttt 4440
agagagaagt atagttttta ttttagtttt gatgttaatt tttgatttat tgttttgttt 4500

taaagtgttt gaataaaatt tgtttgaaaa attattaatg aattaattaa ggtggtgatg 4560
gaaagaaaag ttttgtggtt tttttttttt tgagtaaaga ggtagttttg ttattacgtc 4620
gaattggaat aggaataatt tattttgtat agatttttgtt ggttaggttt tttagattaa 4680
ttaattaggg aatttgtttt ttggtagtgg gaaggtttaa aagaattggg agattttttt 4740
ttgtttttga aagtagagaa gtttgtggtt tatatagcgt gtttttaggt aacgtttaga 4800
aatggaattg agaaatgttt gttttttttt acgtattttt gtttattaag ttaggattga 4860
aagcgaagga aagtttttaa agtttatatt gaatttaatt aaaaattttt aaagatatag 4920
tttgaggatt tttttttta agttttgatt ttgttataga tttttttgt tttttatgtt 4980
tgatatttta gtatttatta atgatttaaa aggttatgta tcggttaggc gaggtggttt 5040
acgttagtaa ttttagtatt ttggaagatc gaagtgggta gattatttga ggttaggagt 5100
ttaagattag tttggttaat atggtgaaat tttgtttttt ttaaaaatat aaaattagtt 5160
agtatgacgg gcgcgtattt ataattttag ttatattgga ggttgaggta ggagaatcgt 5220
ttgaattcgg gaggtagagg ttgtagtgag ttgagatttt tttattgtat tttagtttgg 5280
gcgataagag taaaatttta ttttaaaata aataaataaa tataaataaa aaataataaa 5340
agattaagtt tttaatttag ggtttttgta gaaaggtttt aatgtattgt tttatttatt 5400
gttttatttt aaaattattt aatttaggtt atgttattat ttagtttggt ggttttgggt 5460
aagatagttt tttttggttt tatttttttt atttgtaaaa tgaagagttt gggtaggatg 5520
gtttttttagg tttggtttat tttcgatttt gtatgatttt gagtagaaga agattttttta 5580
gtttcgagtt taagttttat aggtttttta tttcgttttt tgaagagtta agtatattat 5640
aatgttataa gataaaggac gatttttagt ttttttttgt ttttgtttta gaatataaat 5700
ttatggatag tagagtttat agtttttatt tatttgttta gttacgtgtt aacgttttaa 5760
gtattttgat aaaacgtttt taaaatttta gaaataaatt ttggaaagaa atattttttt 5820
aaacgggata aaaatgaatt cggaggttgg gtatggatgg tggtttatgt ttgtaatttt 5880
ttagtatttt gggaggttgg cggattattt gaggttagga gttggagatt agtttgatta 5940
atattaaaaa tataaaaatt agttaggtat gatggtgggt gtttgtagtt ttagttattc 6000
gtgaggttga ggtaaaagaa tttttgatt cggagaggta gaggttgtag tgagttaaga 6060
ttgggttatt gtatttttgt ttggataggg cgagatagcg 6100

<210> 6
<211> 25
<212> DNA
<213> Artificial

<220>
<223> primer

<400> 6
taaggagtaa ggagttttag gggtt 25

<210> 7
<211> 25
<212> DNA
<213> Artificial

<220>
<223> primer

<400> 7
cctaaattta actattattt actaa 25

<210> 8
<211> 25
<212> DNA
<213> Artificial

<220>
<223> primer

<400> 8
atttcctcc ccctaaattt aacta 25

## Description

The present invention provides a method for diagnosis or prognosis of a cancer and a method for prediction of the responsiveness of certain tumor types, said methods comprising determining the methylation degree, i.e. the detection of methylated CpG sites in of the *DUSP4*/*MKP2* gene/promoter. The invention further provides a method for determination of the methylation degree of the promoter region of the dual-specificity phosphatase 4/map kinase phosphatase 2 (*DUSP4*/*MKP2)* gene, which is suitable for prediction of the responsiveness of certain tumor types and for diagnosis or prognosis of cancer. Amplification products and primers which may be used in said method and kits for performing the method are also provided.

## Background of the Invention

Mitogen-activated protein kinase phosphatase 2 (MKP2) is a dual threonine/tyrosine phosphatase that specifically dephosphorylates and inactivates MAP kinases (Misra-Press et al., 1995). MKP2 selectively dephosphorylate both threonine and tyrosine residues of activated ERK1 and ERK2 (Guan et al., 1995). The mechanism whereby MKP2 is regulated and its role during tumorigenesis are largely unknown. Recent findings demonstrated that MKP2 is a novel transcriptional target for E2F-1 that is necessary for the induction of cell death by oxidative stress. E2F-1 binds to a palindrome motif in the promotor and activates the MKP2 promotor, leading to significant transcription of MKP2. Another recently identified palindrome sequence in the MKP2 promoter is a new binding site for p53 to activate the *MKP2* gene. MKP2 is induced prominently when apoptosis occurs and particulary under oxidative stress. Interestingly, MKP2 can induce apoptosis even in the absence of p53, which highlights the independent role of MKP2 in signaling apoptosis (Shen at al., 2006). MKP-2 is located on chromosome 8p12-p11. This locus often shows loss of heterozy-gozity in carcinomas of the breast (Armes et al., 2004; Venter et al., 2005). In brain tumors LOH on chromosome 8p was found in 5% of AII, 14% of AIII, 13% of primary GBM, 4% PA, 7% OA, MI, 50% Metastase (von Deimling et al., 2000). The above human dual-specificity phosphatase 4/map kinase phosphatase 2 (*DUSP4*/*MKP2)* gene is hereinafter shortly referred to as "DUSP4 gene".

Methylation of the *DUSP4* occurs at CpG sites, i.e. at sites wherein C and G are connected by a phosphodiester bond (see Figs. 6a-e). The cytosine in the CpG site is methylated resulting in 5-methylcytosine. The *DUSP4* promoter contains CpG islands, i.e. regions with at least 200 bp which have a GC percentage that is greater than 50% and an observed/expected CpG ratio greater than 0.6 (Gardiner-Garden, M. and Frommer, M., J. Mol. Biol. 1987; 196(2): 261-282). CpG islands are often located around the promoters of frequently expressed genes like housekeeping or repair genes and are usually unmethylated in normal tissues.

Whether a CpG position has been methylated or not can be determined by bisulfite derivatization of the DNA. Cytosine and its methylated counterpart 5-methylcytosine show a different behavior to the treatment with sodium bisulfite on single-stranded DNA. While cytosine residues react with this reagent and are converted to uracil, 5-methylcytosine stays inert under the same conditions. In a subsequent PCR reaction the uracil residues are transcribed to thymine and 5-methylcytosine to cytosine. After cloning and sequencing of the amplicons a comparison with the genomic sequence reveals that a formerly unmethylated CpG-dinucleotide appears as a TpG whereas a methylated one remain as a CpG. Thus, methylated cytosine can be discriminated from unmethylated cytosine. Determination of CpG methylation in the *MGTM* promoter has proved as a useful tool in the diagnosis and prediction of lung cancer (Palmisano, WA et al., Cancer Res. 2000, 60:5954-5958).

Most of the publications dealing with the detection of promoter methylation use a variant of methylation-specific PCR (MSP) (Herman, J.G. et al., Proc. Natl. Acad. Sci. USA 93:9821-26 (1986); Derks, S. et al., M. Cell Oncol. 26: 291-99 (2004); Palmisano et al., loc. cit.*)*. MSP is an important bisulfite-based method for analyzing the DNA methylation status with high sensitivity. This method represents a non-quantitative, cost-efficient analysis. However, MSP bears a significant risk of false positive or false negative results, especially when DNA quality and/or quantity is low, which is often the case in a clinical setting where samples are typically obtained from FFPE (formalin-fixated paraffin-embedded) specimen.

MSP is limited by several constraints. First, mosaic methylation patterns with variable grades of methylation at the primer position can lead to false positive or false negative results. This mis-priming risk further increases when high numbers of PCR cycles or nested primers are used (Derks, S. et al., M. Cell Oncol. 26:291-99 (2004)) as is typically necessary for processing DNA from FFPE samples by MSP.

Second, reliable presence or absence of the methylation-specific band depends on several factors that are difficult to guarantee in a clinical setting, such as reproducible amounts of bisulfite-treated DNA used for amplification and use of high-quality genomic DNA for bisulfite treatment. Third, due to multiple factors influencing band strength (such as PCR performance of each MSP reaction and concentration of bisulfite-treated DNA) MSP cannot provide reliable quantitative information on promoter methylation. In conclusion, the use of MSP in a clinical setting is associated with significant uncertainties and accuracy risks inherent in the PCR reaction, especially if applied to a mosaicism methylation target from a heterogeneous cell population. Therefore it should be used with great caution especially if a therapeutic decision is desired. Hence, problems reported by Hegi et al., N. Engl. J. Med. 352:997-1003 (2005) especially on FFPE tumor samples (for only 67% of the samples the *MGMT* methylation status could be determined by MSP) are not unexpected and further difficulties mentioned in a recent paper (Shaw, R.J. et al., Nucl. Acids Res. 34:e78 (2006)) substantiate the conclusion that MSP is unsuitable for establishment of a robust methylation marker for clinical settings.

Alternative techniques for methylation analysis like bisulfite sequencing of several clones, which are more tolerant towards low sample quality than MSP, are semi-quantitative and widely used in basic research setting but are neither cost effective nor fast enough to be implemented for routine clinical diagnosis.

Bisulfite sequencing of single clones is a widely used application to analyze the methylation profile of an amplicon. Its main advantage is the possibility to analyze each single CpG position with high resolution at the level of single alleles. This results in a quantitative methylation profile of the analyzed region when multiple clones are analyzed. However, the experimental procedure of bisulfite sequencing is a time-consuming and expensive task. Furthermore, subsequent processing of the obtained DNA methylation data is tedious and error-prone work, which requires an experienced experimenter. The typical number of analyzed sequences is in the range of 10 to 15, which can lead to instable means for mosaic methylation patterns and high standard deviations.

Thus, the problem underlying present invention is the provision of an improved method for the determination of *DUSP4* promoter methylation which does not possess the drawbacks described above for known methods and which is suitable for application in a clinical setting. The *DUSP4* promoter methylation degree may be used as an epigenetic marker for cancer diagnosis and cancer prediction, and for prognosis of the effect of alkylating agents on a cancer.

**Summary of the Invention**

It was found in a genome-wide screen using differential methylation hybridization that a CG-rich segment within the promoter region of the *dual-specificity phosphatase 4*/*map kinase phosphatase 2 gene* (*DUSP4*/*MKP2*) that showed hypermethylation in gliomas compared to normal white matter controls. Bisulfite sequencing and COBRA was performed to confirm the methylation status of this CpG island in 96 gliomas and 7 glioma cell lines. Hypermethylation was detected in 4/43 (9%) primary glioblastomas. 11/13 (85%) secondary glioblastomas, 5/9 (56%) anaplastic astrocytomas, 5/7 (71%) low-grade astrocytomas, 6/6 (100%) OII, 4/5 (80%) AOIII, 3/3 (100%) AOAIII and 5 out of 7 (71%) glioma cell lines. None of three investigated white matter samples showed hypermethylation of the *DUSP4* promoter region. A significant correlation was found between methylation of *DUSP4* and prolonged survival of glioblastoma patients. Expression of *DUSP4* was investigate by real-time reverse transcription-PCR and found to be significantly downregulated in secondary GMB and anaplastic and low-grade astrocytomas when compared to normal brain controls. After treatment of glioma cell lines with 5-aza-2'-deoxycytidine, transcription of *DUSP4* was restored. The frequent methylation of *DUSP4* in secondary glioblastomas and the rare alterations found in primary glioblastomas is intriguing because they represent two clinically distinct glioblastoma entities. Moreover, the inactivation of the MAP kinase regulator *DUSP4* in secondary glioblastomas and the established amplification *EGFR* in primary glioblastomas point to a major contribution of the MAP kinase pathway in the molecular pathogenesis of astrocytic gliomas. Recent studies demonstrate that the putative tumor suppressor gene *DUSP4* is a primary target gene of p53 and required for the induction of apoptosis following oxidative stress. Taken together these results argue for a role of *DUSP4* in the development of gliomas and may point to novel therapeutic targets for glioblastomas that do not contain an amplified or mutant EGFR receptor gene. In other words, the methylation of the promoter region of DUSP4 was identified as molecular marker for gliomas. Also in mamma carcinomas and metastases a methylation *of DUSP4* could be identified.

Furthermore a robust, cost-efficient, and easy-to-use method for the sensitive and quantitative assessment of *DUSP4* methylation on isolated DNA, especially genomic DNA was found. Its general applicability was confirmed on DNA extracted from frozen tissues and formalin-fixed, paraffin-embedded (FFPE) specimens. The method is preferably performed using Pyrosequencing™ or COBRA, more preferably using Pyrosequencing™.

Hypermethylation of the *DUSP4* gene alters DNA repair and is associated with longer survival of glioblastoma patients treated with alkylating agents. Methylation of *DUSP4* is also found in several other cancer entities. Therefore *DUSP4* plays an important role as a predictive epigenetic marker for chemotherapy resistance. To adopt this established correlation into a molecular diagnosis and prognosis procedure, we compared different technologies and different CpG sites with regard to their accuracy in reflecting the *DUSP4* methylation.

Sodium bisulfite treatment to modify genomic DNA from 96 gliomas and 7 glioma cell lines and 3 white matter biopsies as normal brain controls. The bisulfite derived PCR products were further analyzed by either sequencing of individual clones, or by COBRA to investigate altered methylation *of DUSP4.*

Most informative CpG positions in the promoter region of *DUSP4* were identified discriminating specific glioma DNA. The results of this comprehensive investigation were used for the development a COBRA assay to assess the methylation state at these positions. Due to the reliable (no false positives/negatives), quantitative and sensitive detection of *DUSP4* methylation in the method of present invention, its outcome is a reliable basis for decisions on suitable therapy for several tumor entities.

Thus, the present invention provides

(1) a method for determination of the methylation degree of the dual-specificity phosphatase 4/map kinase phosphatase 2 (*DUSP4)* 5'non-coding region (*"DUSP4* promoter") in a DNA sample, which comprises

(a) treating the DNA sample with bisulfite;
(b) amplifying a segment ("amplified segment") of the bisulfite-modified DNA obtained in step (a) by PCR using the bisulfite treated DNA as template, wherein the amplified segment comprises at least 20 consecutive bisulfite-modified nucleotides within a 5' untranslated region of the native *DUSP4* sequence represented by bp 1 to 8276 of SEQ ID NO:1 (i.e. 5'to the ATG at 8277-9 of SEQ ID NO:1) and spans at least three CpG sites ("selected CpG sites"); and
(c) analysing the amplification product and determining the methylation status of the selected CpG sites in the amplified segment;

(2) the method according to embodiment (1) above, wherein the 5' untranslated region is (i) bp 705 to 1006 of SEQ ID NO:1 (which corresponds to bp -7571 to - 7270 of the sequence of NM_057158 (NCBI)); or (ii) the selected CpG sites covered by the amplified fragment comprise either CpG 3 and 4 to CpG 8 and 9 or (ii) CpG 8 and 9 to CpG 14 and 15;

(3) the method of embodiment (1) or (2) above, wherein step (c) comprises Pyrosequencing™ or restriction analysis of the amplification product;

(4) a method for prediction of the responsiveness of tumors to agents acting on the amplified/mutated EGFR gene which comprises determining the methylation degree of the *DUSP4* gene, preferably that of the *DUSP4* promoter in a DNA sample, wherein the methylation degree of selected CpG sites in the *DUSP4* gene/promoter is indicative for the responsiveness of the tumor, most preferably the method of any one of embodiments (1) to (3) above is applied;

(5) a method for diagnosis or prognosis of a cancer which comprises determining the methylation degree of the *DUSP4* gene, preferably that of the *DUSP4* promoter in a DNA sample, wherein the methylation degree of selected CpG sites in the *DUSP4* gene/promoter is indicative for a cancer or cancer risk, most preferably the method of any one of embodiments (1) to (3) above is applied;

(6) an isolated DNA molecule which is

(i) the amplification product as obtainable by step (b) according to any one of embodiments (1) to (3) above;
(ii) a primer for the PCR reaction in step (b); or
(iii) a primer for the Pyrosequencing reaction of embodiment (3) above; and

(7) a kit for performing the method of any one of embodiments (1) to (5) above.

**Short Description of the Figures**

Fig. 1. Identification of the methylation of DUSP4 in diffuse astrocytomas. (a) Detail of a CpG island microarray hybridized according to the DMH protocol with genomic DNA of a secondary glioblastoma and white matter; (b) BLAST result of the identified CpG rich fragment using the UCSC genome browser; (c) CG-rich DUSP4 promoter segment (SEQ ID NO:2).
Fig. 2. Summary of bisulfite sequencing analysis of CpG sites 1 to 23 of individual clones in gliomas and white matter control. Black circle, methylated CpG site, white circle unmethylated CpG site. d: COBRA analysis of the 5'-UTR of DUSP4. The 363 bp bisulfite PCR product was digested with the *BstU*1 restriction enzyme. Restriction fragments are only apparent in gliomas and not in normal white matter samples (B1-3).
Fig.3.(a) Location of the *BstU*I restriction sites within the amplified region relevant for the COBRA assay; (b) Kaplan-Meier curve showing significant correlation between DUSP4 methylation (red line) and prolonged survival; (c, d) separation of restriction fragments after *BstU*I digestion in primary glioblastomas (c) and astrocytomas WHO grade II (d).
Fig. 4. Correlation between expression and methylation of DUSP4 in relevant glioma entities.

Fig. 5.(a) Relative transcript levels of DUSP4 in glioma cell lines with or without µM 5'aza-deoxycytidine treatment; (b) Expression of DUSP4 analyzed by RT-PCR after transfection of U373MG cells with DUSP4-pcDNA4.1 Myc/his plasmid or pcDNA4.1 My/his empty vector. (c) (d) (e).

Fig. 6 (a) + strand of the native 5' untranslated region of the DUSP4 gene, the preferred primer sequences and the ATG start-codon of DUSP4 are boxed and the preferred CGs are numbered (the sequence corresponds to SEQ ID NO:1); (b) native reverse complement of the DUSP4 sequence of SEQ ID NO:1 (SEQ ID NO:3), with highlighted CG-sites and primers in capital letters; (c) modified reverse complement of the DUSP4 sequence of SEQ ID NO:1 (non-methylated) (SEQ ID NO:4) with highlighted TG-sites; (d) modified reverse complement of the DUSP4 sequence of SEQ ID NO:1 (methylated) (SEQ ID NO:5) with highlighted CG-sites, boxed amplicon and primers in capital letters; (e) native reverse complement of the DUSP4 sequence of SEQ ID NO:1 (SEQ ID NO:3) with restriction sites.

**Sequence Listing - Free Text**

| SEQ ID NO: | Designation |
|---|---|
| 1 | part of human *DUSP4* comprising the *DUSP4* promoter |
| 2 | pyrosequenced segment of SEQ ID NO:1 |
| 3 | Non-modified reverse complement of SEQ ID NO:1 |
| 4 | modified reverse complement of SEQ ID NO:1 (non-methylated) |
| 5 | modified reverse complement of SEQ ID NO:1 (methylated) |
| 6 | DUSP4 fw primer |
| 7 | DUSP4 rev primer |
| 8 | DUSP4 rev primer |

**Detailed Description of the Invention**

The present invention provides an improved analysis method of *DUSP4* promoter methylation for application in a clinical setting as an epigenetic marker, preferably an analysis metod for secondary glioblastomas and low-grade gliomas/astrocytomas (diffuse astrocytomas WHO grade II und anaplastic astrocytomas WHO Grad III), which do not contain an amplified/mutated EGFR gene and can consequently not be treated with therapeutics directed against this epitope. SEQ ID NO:1 represents a segment of the native, unmethylated *DUSP4* gene and its 5'regian, The determined region of DUSP4 corresponds to nt -7571 to 7270 of NCBI accession number NM057158 (the relevant portion thereof is shown in SEQ ID NO:1). The CpG positions which are relevant for the present invention are located in SEQ ID NO: 1 as follows:

CpG 01: bp 709 + 710; CpG 02: bp 715 + 716; CpG 03: bp 722 + 723;
CpG 04: bp 724 + 725; CpG 05: bp 730 + 731; CpG 06: bp 733 + 734;
CpG 07: bp 742 + 743; CpG 08: bp 779 + 780; CpG 09: bp 781 + 782;
CpG 10: bp 786 + 787; CpG 11: bp 790 + 791; CpG 12: bp 801 + 802;
CpG 13: bp 818 + 819; CpG 14: bp 823 + 824; CpG 15: bp 825 + 826;
CpG 16: bp 838 + 839; CpG 17: bp 912 + 913; CpG 18: bp 915 + 916;
CpG 19: bp 926 + 927; CpG 20: bp 946 + 947; CpG 21: bp 949 + 950;
CpG 22: bp 977 + 978; CpG 23 : bp 1005 + 1006.

The *DUSP4* promoter CpG island to which the following part refers is represented by nt 401 to 1200 of SEQ ID NO:1.

The term "5'non-coding region" has several synonyms, namely "*DUSP4* promoter" and *"DUSP4* promoter region", which are used throughout the present invention. These terms do all designate the 5'region of the *DUSP4* gene which does control *DUSP4* transcription and whose effect on transcription can be influenced by CpG methylation. In the context of present invention, the *PUSP4* promoter consists of the sequence represented by SEQ ID NO:1.

In the following, bp and nt will be used synonymously for indication of positions in a nucleic acid sequence. Therefore, a position indication for double stranded DNA by nt always includes the basepair and the complementary nucleotide on the complementary strand.

The amplification reaction in step (b) of embodiment (1) is performed using the bisulfite-modified DNA obtained by the bisulfite treatment of the initial DNA sample in step (a). The outcome of the bisufite treatment depends on whether a C was methylated or not in the initial DNA sample. Thus, the nucleotide sequence of the bisulfite-treated DNA will differ from the bisulfite-untreated DNA sequence: every unmethylated C has reacted with bisulfite and was converted to uracil, every 5-methylcytosine stayed inert under the same conditions. In a subsequent PCR reaction the uracil residues are transcribed to thymine and 5-methylcytosine to cytosine. As the number of methylated Cs in the samples will differ from sample to sample (generally, the Cs in DNA samples from healthy persons are generally unmethylated, whilst a considerable portion of the Cs in DNA samples from tumors is methylated), there are more than one possible nucleotide sequences resulting from the bisulfite treatment of the *DUSP4* gene. Thus, in order to simplfy comparison, the nucleotide positions in DNA sequences resulting from the bisulfite treatment ("bisulfite treated counterpart" of the initial DNA) and from PCR amplification reactions perforemd on said bisulfite-treated counterpart will be given as the corresponding positions in the native *DUSP4* sequence represented by SEQ ID NO:1. The tern "bisulfite-treated counterpart" within the ambit of the invention also includes the sequence complementary to said counterpart, i.e. the amplification product of both bisulfite treated strands. It should be kept in mind that in the bisulfite-treated DNA the C may be replaced by a U and that in the amplification products the CG basepair at the site of such U is replaced by TA.

"Selected CpG sites" in the context of present invention contribute to the determination of methylation status of the *DUSP4* gene because they are analyzed in step (c) in order to establish whether they were methylated or not.

In a preferred aspect of embodiment (1), the at least three selected CpG sites covered by the amplified segment are selected from the group consisting of the CpG sites at positions CpG 1 to 23. More preferably, they are selected from the group consisting of CpG 3 to 15.. Most preferably, they are (i) CpG 3 to 9. The amplified segment may span additional CpG sites.

In a further preferred aspect of embodiment (1), the amplified segment in step (b) spans at least 4 selected CpG sites.

Moreover, ist is preferred that the amplified segment comprises at least 20 consecutive nucleotides of the bisulfite-treated counterpart of the sequence represented by nt 241 to 6129 of SEQ ID NO:1. More preferably, is comprises at least the bisulfite treated counterpart of the sequence represented by nt 681 to 1032 or nt 1392 to 1890 of SEQ ID NO:1.

The amplification step (b) of embodiment (1) is performed by PCR. The primer pair ("first primer pair") used in said PCR ("first PCR") does preferably not amplify bisulfite-untreated genomic DNA. In other words, the primers or at least one of the primers are complementary to a segment of SEQ IN NO:1 after the bisulfite treatment of said segment ("bisulfite treated counterpart"), but not beforehand. Such primers are selective for the bisufite-treated region of interest of SEQ ID NO:1.

The first primer pair consists of a forward and reverse primer. In a preferred aspect of embodiment (1), the forward primer is complementary to at least 10 consecutive nucleotides of nt 681 to 705 of SEQ ID NO:1. or its bisulfite-treated counterpart, and the reverse primer is complementary to at least 10 consecutive nucleotides of nt 1007 to 1032 of SEQ ID NO:1 or its bisulfite-treated counterpart. Preferably, it is complementary to the bisulfite counterpart of the segments listed in the previous sentence, more preferably it binds exclusively to said counterpart.

In any of embodiments (1) to (3), the method may comprise an additional step after step (c), namely a statistical analysis of the methylation scores for the selected CpGs. This allows the separation of DNA samples containing unmethylated *DUSP4* from DNA samples containing methylated *DUSP4.* It furthermore provides a confidence estimate, i.e. shows the reliability of the methylation prediction. Analysis of selected CpG sites 4, 5, 8, 9, 10, 14 and 15 are the most reliable means for determination of *DUSP4* methylation.

The statistical analysis is performed using the following classification formulae(e.g. for CpGs 1, 2, 5, 8 and 9):

CpGs 1, 2, 5, 8 and 9:

$$Score = -339.385 \cdot CpG_{1and2} + 196.192 \cdot CpG_{8and9} + 137.296 \cdot CpG_5 - 21.803\ ;$$

CpGs 9 to 12:

$$Score = 21.330 \cdot CpG_9 + 24.806 \cdot CpG_{10} + 18.637 \cdot CpG_{11} + 21.503 \cdot CpG_{12} - 20.197\ ,$$

where CpG variables refer to the measured methylation score at each position. Overall positive scores predict promoter methylation, negative scores the absence of promoter methylation. A an unexpected negative coefficient at CpG position 1/2 was observed, a high methylation level at this position is not a good predictor of a high overall methylation level.

For all formulae, the resulting score for any single tumor not only gives a prediction of the overall promoter methylation state but also provides a confidence estimate: the further the score deviates from zero, the safer is the prediction.

For CpGs 1, 2, 5, 8 and 9 scores below -15 are highly indicative of overall absence of methylation, while scores above 40 are consistently associated with overall promoter methylation. Between these safety margins, no clear conclusion is possible. For CpGs 9 to 12, safe prediction of unmethylated promoter state is possible below -10, while methylation is present at scores above 10.

It is to be noted that the known method for determination of *DUSP4* methylation, MSP, is not focussed on CpG 9 to 12, but on other regions of the *DUSP4* gene. Especially CpG 1, 2, 5, 8, 9, 10, 11 and 12 differ from the positions known as indicative for *DUSP4* promoter methylation in MSP. Furthermore, MSP does not allow a quantitative determination of the methylation degree.

One aspect of present invention is the method of embodiment (3), wherein the analysis step (c) is performed using Pyrosequencing. This analysis method allows the simultaneous analysis of several CpG positions in a range of up to 30 bp and which generates quantitative results for each individually analyzed CpG position. Rapid and parallel processing of a large number of samples is a very important advantage of this technology.

The Pyrosequencing method is limited due to the need of an appropriate primer for extension. The Pyrosequencing primer is preferably designed to minimize the amount of not properly bisufite-converted alleles. This is achieved by incorporating a bisulfite-conversion specific thymine residue on the 3'-end, which helps to avoid mis-priming of residual unconverted DNA which may lead to a false positive interpretation.

In the method using Pyrosequencing, step (b) comprises only one PCR amplification reaction. This minimizes the time, equipment and effort necessary for performing the method, especially in comparison to MSP.

The method using pyrosequencing is preferably performed with an amplified segment comprising at least 20 consecutive nt of the bisulfite-treated counterpart of SEQ ID NO:1. More preferably, the segment has a maximum length of 200 nt, even more preferably of up to 100 nt. However, a segment with 20 nt length is sufficient.

Furthermore, the amplified segment preferably comprises or consists of the sequence represented by nt 1086 to 1105 of the bisulfite-treated counterpart of SEQ ID NO:1.

Finally, in the method using Pyrosequencing, the selected CpG sites comprise at least three CpG sites selected from CpG 9 to 12, and more preferably comprise all 4 CpG sites CpG 9 to 12. The analysis of the methylation status of CpG 9 to 12 (and no further CpGs) is sufficient for a reliable statement on the methylation of the *DUSP4* promoter. It is even sufficient for a reliably determination of the methylation status of the *DUSP4* when CpG 9 to 12 are the only CpGs whose methylation status is determined. However, if three of the four sites are methylated or if three of the sites are not methylated, this also leads to a reliable statement. Thus, the analysis of three CpG sites selected from CpG 9 to 12, especially of CpG 9 to 11, is also preferred.

There are several preferred aspects of the method using Pyrosequencing: In one preferred aspect, in the primer pair used in the amplification step (b) the forward primer is complementary to at least 10 consecutive nucleotides of nt 681 to 706 of SEQ ID NO:1 or its bisulfite-treated counterpart, and the reverse primer is complementary to at least 10 consecutive nucleotides of nt 1007 to 1 032 of SEQ ID NO:1 or its bisulfite-treated counterpart. Preferably, it is complementary to the bisulfite counterpart of the segments listed in the previous sentence, more preferably it binds exclusively to said counterpart.

The second especially preferred aspect of present invention is the method of embodiment (3), wherein the analysis step (c) comprises a restriction digestion of the amplification product and subsequent analysis of the restriction products. A synonym for this method in the context of present invention is COBRA ((***Co***mbined ***B***isulfite ***R***estriction analysis). However, of the two methods of embodiment (3), the method comprising the Pyrosequencing analysis is preferred.

In COBRA, the selected CpG sites preferably comprise at least two pairs out of CpG 3 and 4, CpG 8 and 9 and CpG 14 and 15, preferably comprise CpG 3, 4, 8, 9, 14 and 15. The analysis of the methylation status of CpGs 3 and 4, and 8 and 9 or of CpGs 8 and 9, and 14 and 15 (and no further CpGs) is sufficient for a reliable statement on the methylation of the *DUSP4* promoter. However, as the use of certain restriction endonucleases (like *BstUI* used in the examples section) which cut at CpG 3/4, 8/9 and 14/15, a longer amplification segment inevitably leads to cuts at al of said positions, the selected CpG sites preferably comprise CpG 3, 4, 8, 9, 14 and 15.

Furthermore, COBRA preferably comprises in step (b) one PCR reaction: It is performed with a first primer pair using the bisulfite-treated template obtained from step (a). The second PCR reaction is performed with a second primer pair using the amplification product of the first PCR reaction and a second primer pair. The first primer pair contains 5'-tails of at least 10 nt length which are complementary to each other and identical to the two second primer sequences. In the second primer pair, one primer is labelled at its 5'tail with one dye molecule (first label) and the second primer is labelled at its 5'tail with a different dye molecule (second label). The first and/or second label allow direct optical detection, such as by detection of fluorescence, chemiluminescence or the like, provided that said second detectable label differs from said first detectable label in order to allow separate detection.

In the primer pair used in the first PCR of COBRA, the forward primer is preferably complementary to at least 10 consecutive nucleotides of nt 681 to 705 of SEQ ID NO:1 or its bisufite-treated counterpart, and the reverse primer is preferably complementary to at least 10 consecutive nucleotides of nt 1007 to 1032 of SEQ ID NO:1 or its bisulfite treated counterpart. Preferably, it is complementary to the bisulfite counterpart of the segments listed in the previous sentence, more preferably it binds exclusively to said counterpart. Most preferred primers of the first primer pair are selected from the group consisting of the sequences represented by SEQ ID NOs:6, 7 or 8, sequences comprising SEQ ID NOs:6, 7 or 8, and also sequences comprising or consisting of SEQ ID NOs:6, 7or 8, which are truncated by up to three nucleotides at their 5' and/or 3'-end.

The primers of the primer pair used in the second PCR ("second primer pair") are preferably without binding site in the initial DNA sample. More preferably they are also without binding site in the bisulfite-treated counterpart of the initial DNA sample or the amplification product thereof. Thus, they do only bind to each other in the second PCR. Suitable second primer pairs are the non-matching M13 tail primer pair (SEQ ID NOs:6, 7 and 8; derived from bacteriophage M13) or primer pairs comprising at least 10 consecutive nucleotides thereof. Moreover, the primers of the second primer pair are preferably at least 13 nt long.

It is also preferred that the labels at the 5'ends of the second primer pair are state fluorescent or luminescent dye molecules for DNA as known in the art, preferably fluorescein derivatives, more preferably FAM (6-Carboxyfluorescein) and/or JOE (2,7-dimethoxy-4,5-dichloro-6-carboxyfluorescein).

The COBRA method is preferably performed with an amplified segment comprising at least 48 consecutive nt of the bisulfite-treated counterpart of SEQ ID NO:1. More preferably, the segment has a maximum length of 200 nt, even more preferably of up to 100 nt. However, a segment with 48 nt length is sufficient.

Furthermore, the amplified segment preferably comprises or consists of the sequence represented by nt 706 to 1006 of the bisulfite-treated counterpart of SEQ ID NO:1.

Step (c) in COBRA is preferably performed on the amplification product of the second PCR reaction described above. The amplification product which is analyzed in step (c) is preferably labelled with two dye molecules which are different from each other, wherein one dye molecule is covalently linked to the 3'end and the other to the 5'end of the amplification product. More preferably, said amplification product is the result of the second PCR in step (b) using the labelled primers as defined above for the second primer pair.

Finally, in a further preferred aspect of COBRA, at least one restriction endonuclease used in the restriction digestion of step (c) has a recognition site which spans at least one of the selected CpG sites and does recognize only a recognition site containing CpG at the CpG site in the amplification product obtained by step (b). The recognition sites of the endonucleases are preferably comprising CpG 3, 4, 8, 9, 14 and/or 15 or their counterparts resulting fro the bisulfite treatment and the subsequent PCR amplification (see Fig 6a). More preferably, they comprise one of said CpG and one, two, or three flanking nucleotides on one or on each side of the CpG. Thus, they most preferably are located in nt 720 830 of SEQ ID NO:1. Utmost preferred are restriction endonucleases having recognition sites comprising or consisting of TCGA or CGCG, including Taq"I and BstUI (which are the most preferred), Bst1236I (FmuDII), AccII, BstFNI, MvnI (the latter four cut like BstUI) and TaqI (cuts like Taq$^{\alpha}$I).

The DNA sample used in embodiments (1) to (3) is preferably genomic DNA. The method according to the invention is so robust that the use of this rather low quality DNA which comprises mostly non-*DUSP4*-sequences is tolerated and does not falsify the result.

The DNA sample is preferably isolated from mammalian, more preferably from human tissue. Any mammalian DNA can be used in the method according to the invention, but of course human DNA is of the utmost clinical interest. Furthermore, said tissue may be freshly prepared (e.g. is a biopsy sample) or may be a sample which has been stored before application of the method of embodiment (1). Notably, the method allows the determination of the *DUSP4* methylation grade in kryopreserved or formalin-fixed tissue. This is advantageous, as it allows statements on samples which were not kryopreserved, were stored in pathological collections and archives or were taken in the far past.

The DNA sample is in a further preferred aspect of embodiment (1) isolated from a tumor tissue. Preferably, said tumor tissue is tissue isolated from glioma (including glioblastoma multiforme, low grade astrocytoma gliobastoma, oligodendroglioma), diffuse large B-cell lymphoma (DLBLC), colorectal carcinoma, non-small cell lung carcinoma, lymphoma, head and neck carcinoma and brain tumors (including anaplastic astrocytoma, medulloblastoma, neuroblastoma), more preferably tumor tissue isolated from glioma, colorectal carcinoma and DLBLC, most preferably tumor tissue isolated from malignant glioma, especially glioblastoma, and DLBLC.

In a preferred aspect of embodiment (4), a methylation degree of 75 % or more, preferably of 90 % of the selected CpG sites indicates that the tumor is susceptible to alkylating agents.

Embodiment (5) allows prognosis and diagnosis of cancer by the method of any of embodiments (1) to (3). However, any DNA analytic method allowing the discrimination between methylated and unmethylated CpG 9 to 11, preferably CpG 9 to 12, e.g. hybridisation with appropriate probes, is suitable as the mandatory first step towards a reliable diagnosis/prognosis.

The primer of embodiment (6)(ii) is any primer of the first primer pair or second primer pair as described above. In a preferred aspect of embodiment (6)(ii), the primer is complementary to at least 10 consecutive nucleotides of nt 681 to 725 or 1007 to 1032 of SEQ ID NO:1 or the bisulfite-treated counterpart of SEQ ID NO:1.

The primer may comprise a tail as described above and exemplified for M13, and it may be labeled with a label as outlined above.

The most preferred primers of embodiment (6)(ii) are selected from the group consisting of the sequences represented by SEQ ID NOs:6, 7 and 8, sequences comprising SEQ ID NOs:6, 7 or 8 and also sequences comprising or consisting of SEQ ID NOs:2 or 3 which are truncated by up to three nucleotides at their 5' and/or 3'-end.

The Pyrosequencing primer of embodiment (6)(iii) is preferably a primer as outlined above as suitable for the preferred pyrosequenced segment, more preferably a primer selected from the group consisting of the sequence represented by SEQ ID NO:8, sequences comprising SEQ ID NO:8 and also sequences comprising or consisting of SEQ ID NO:8 which are truncated by up to three nucleotides at their 5' and/or 3'-end.

The kit of embodiment (7) preferably comprises one or more of the primers of embodiment (6) and/or one or more of the restriction endonucleases indicated as suitable for COBRA above.

Several of the preferred aspects of present invention are described in more detail in the following sections:

Several of the preferred aspects of present invention are described in more detail in the following sections:

Resistance to chemotherapy is a major complication during treatment of cancer patients. Hypermethylation of the *DUSP4*-gene alters DNA repair and is associated with longer survival of glioblastoma patients treated with alkylating agents. Methylation of *DUSP4* is also found in several other cancer entities. Therefore *DUSP4* plays an important role as a predictive epigenetic marker for cancer and for chemotherapy resistance. To adopt this established correlation into a molecular diagnostic procedure, we compared different technologies with regard to their sensitive and reproducible detection of methylation in frozen and paraffin embedded tissues. DNA was extracted either from frozen or formalin-fixed, paraffin-embedded (FFPE) gliobastoma samples. Following bisulfite treatment and PCR, sequencing of individual clones and COBRA was used to investigate altered methylation of *DUSP4.* The most informative CpG positions in the promoter region were identified that discriminate glioblastoma DNA from normal brain tissues. The results of this comprehensive investigation were used for development of different assays to assess the methylation state at these positions. Finally, these approaches were compared with regard to their robustness, significance and reproducibility. Based on the results, a robust, cost-efficient, and easy-to-use clinical diagnosis platform was established that is suitable for the sensitive and quantitative assessment of *DUSP4* methylation as a reliable clinical tool.

Positions in the *DUSP4* promoter were verified that are reliably correlated with the overall methylation state of the promoter and - at the same time - are accessible to bisulfite sequencing and COBRA (Combined ***B***isulfite ***R***estriction ***A***nalysis). Second, each method was systematically optimized for robust determination of *DUSP4* promoter methylation and tested its performance on well-characterized tumor samples. Finally, the results for said three techniques were compared with respect to reliability, expenditure, and applicability for molecular diagnostics.

First, detailed *DUSP4* promoter methylation data were established for 22 tumor samples by bisulfite sequencing data (Fig. 2a,b,c) and two distinct subclasses were observed. The first subclass exhibited entirely unmethylated alleles with sporadic cases of methylated CpG positions (e.g. normal brain white substance Fig. 2a). In contrast, tumors in the second subclass demonstrated high and variable methylation levels (e.g. Sample 96-98, Fig. 2b). Alleles were on average densely methylated but with variation between CpG positions and between different alleles.

Restriction-based methods such as COBRA are typically used when the methylation profile is known and the most informative positions or regions in an amplicon have been identified. With the help of restriction endonucleases that recognize DNA methylation at specific sites, the informative positions can be analyzed. The recognition of the restriction site and the number of cutting sites are the most limiting factors of this technology because in many cases there is no enzyme available that fulfills the demands for analyzing the position(s) of interest. Furthermore the reaction conditions for a suitable enzyme must be carefully established, to guarantee a complete digestion without any unwanted side effects. On the other hand, when an appropriate enzyme or enzyme-combination is suitable it results in a cost-effective assay to obtain quantitative or semi-quantitative information of the methylation level for the screened CpG position. Another advantage of COBRA is the possibility to process a large series of samples in parallel.

The established COBRA assay is suitable for both high-quality and low-quality DNA material. The chosen system with the restriction endonuclease *BstUI* avoids misinterpretation of the obtained results. The use of this enzyme minimizes false positive results because both cytosine positions within the restriction site have to be methylated to conserve the restriction site during bisulfite treatment. An occasionally sparse methylation will not result in digestion of the PCR product, which contains three *BstU*I restriction sites.

The developed COBRA assay is therefore suitable for use in clinical settings to assess *DUSP4* promoter methylation.

The main focus was to translate the important epigenetic marker *DUSP4* into a robust and easy-to-use clinical diagnosis tool that can be applied to DNA extracted from clinical samples. The marker's robustness was confirmed on FFPE specimen, opening up the possibility to accurately investigate *DUSP4* methylation in archival tissues.

The present invention is described in more detail by reference to the following examples. It should be understood that these examples are for illustrative purpose only and are not to be construed as limiting the invention.

**Examples**

In the following examples, standard techniques of recombinant DNA technology and molecular biology were used that were described in various publications, e.g. Sam-brook et al. (2001), Molecular Cloning: A Laboratory Manual, 3rd edition, Cold Spring Harbor Laboratory Press, or Ausubel et al. (1987), Current Protocols in Molecular Biology, Current Protocols in Immunology, Current Protocols in Protein Science, and Current Protocols in Cell Biology, edition as of 2002, Wiley Interscience. Unless otherwise indicated, all enzymes, reagents, devices and kits were used according to the manufacturers' instructions.

Example 1: Materials and Methods

1.1 DNA samples and cell lines: The glioma cell lines A172, U373MG, LN229, U251MG, U87MG, U178MG and LN314 were cultured in DMEM medium with 10% FCS (Biochrom, Berlin, Germany) supplemented with 4.5g/l glucose and 4mM l-glutamine at 37°C and 5% $CO_2$. A cohort of 86 gliomas and 7 cell lines was analyzed in this study, including representatives of the major histopathological entities (66 primary GBM, 12 sec. GBM, 9 AAIII, 7 AII, 5 OII, 3 OAII, 3 AOIII). All biopsies were diagnosed according to the WHO classification of brain tumors, using standard histological and immunohistological methods (Kleihues et al., 2002 ?). Histological assessment of tissue fragments chosen for this study confirmed that all specimens consisted of at least 80% tumor cells. Tissues were selected for extraction of DNA and RNA after careful examination of H&E staining of corresponding frozen sections to exclude contaminating necrotic debris or normal brain tissue and to determine the histological characteristics of the tumor. DNA was extracted using standard proteinase K digestion and phenol/chloroform extraction. RNA was isolated with TRI-ZOL reagent (Invitrogen, La Jolla, CA) following the manufacturers protocol. Contaminating residual genomic DNA was removed by digestion with RNase-free DNase (Roche, Mannheim, Germany). Biopsies of white matter were included as normal tissue controls for hybridization of the DMH (differential methylation hybridization) microarrays and for methylation and expression analysis.

1.2 Differential methylation hybridization (DMH) analysis: The DMH procedure was performed as described (Huang 1999). CpG-rich DNA fragments were isolated from the human CpG island library and screened for the presence of *BstUI* (New England Biolabs) and *HpaII* (New England Biolabs) restriction sites. A total of 16.000 suitable fragments were PCR-amplified using plasmid primer and spotted in duplicates onto UltraGAPS microarrays (Corning, Acton, MA). For amplicon generation, 2 μg of genomic DNA of 40 glioma samples (10 primary glioblastomas, 10 secondary glioblastomas 10 anaplastic astrocytomas and 10 low grade diffuse astrocytomas) samples and a pool of 3 white matter samples were digested with *MseI* (New England Biolabs). After ligation of linker H12/H24, fragments were digested with methylation-sensitive restriction endonucleases *BstUI* and *HpaII* and amplified for 20 cycles with H24 as primer (AGGCAACTGTGCTATCCGAGGGAT). PCR fragments were indirect labeled with the Alexa Fluor 555 (white matter) and Alexa Fluor 647 (tumor samples) using the BioPrime Plus Array CGH Indirect Genomic Labeling Kit (Invitrogen). Labeled amplicons were purified with delivered colums and equal amounts of Alexa Fluor 555 and Alexa Fluor 647 and 10 μg human Cot-1 DNA (Invitrogen) were combined for hybridization on DMH microarrays. Hybridization and analysis were carried out as described (Yan 2001 ?). Data from single-copy sequences were normalized and loci with a Alexa Fluor 647/ Alexa Fluor 555 ratio greater than 2.5 were scored as hypermethylated.

1.3 Bisulfite treatment: Genomic DNA (300-500 ng) was subjected to bisulfite conversion with the EpiTect® Bisulfite Kit (Qiagen) according to the manufacturer's instructions. Cytosine and its counterpart 5-methylcytosine show a different behavior to the treatment with sodium bisulfite on single-stranded DNA. While cytosine residues reacted with this reagent and are converted to uracil, 5-methylcytosine stayed inert under the same conditions. In a subsequent PCR reaction the uracil residues were transcribed to thymine and 5-methylcytosine to cytosine. After cloning and sequencing of the amplicons a comparison with the genomic sequence revealed that a formerly unmethylated CpG-dinucleotide appears as a TpG whereas a methylated one remained as a CpG.

1.4 Bisulfite sequencing: To analyze the methylation status of DUSP4, genomic DNA of gliomas, cell lines and non-neoplastic white matter tissue was extracted and treated with sodium bisulfite using the EpiTect Bisulfite kit (Qiagen, Hilden, Germany). Primers used for PCR amplification were DUSP4 fw-5'-taaggagtaaggagttttaggggtt-3' (SEQ ID NO:6) and DUSP4 rev-5'-attttcctccccctaaatttaacta-3' (SEQ ID NO:8). The annealing temperature for the bisulfite PCR was 54°C. The 363 bp PCR product contains 23 putative CpG methylation sites. For bisulfite sequencing, the bisulfite PCR products were cloned into the TOPO TA cloning/pCR2.1 vector (Invitrogen). Inserts of individual bacterial clones were amplified using vector-specific primers (fw-ttgggtaacgccagggt and rev- cag-gaaacagctatgac). PCR products were treated with ExoSAP-IT (usb) and sequenced using the BigDye Prism DNA sequencing kit (Applied Biosystems, Foster City, CA). Single clone sequences were analyzed and diagrams were generated with the BiQ Analyzer software (Bock et al.,

2005).

1.5 Combined Bisulfite Restriction Analysis COBRA): PCR products generated with *DUSP4* primers (DUSP4 fw-5'-taaggagtaaggagttttagggggtt-3' and DUSP4 rev-5'-attttcctccccctaaatttaacta-3') from bisulfite treated tumor and control DNA were digested with the restriction enzyme *BSTUI* (NEB) and subsequently separated on agarose gels. *BSTU*I restriction sites (CGCG) are conserved when the respective DNA samples are methylated while unmethylated DNA samples are changed by bisulfite treatment into TGTG and are not recognized by the enzyme. The 363 bp amplicon of *DUSP4* (corresponding to nt... to ... of SEQ ID NO:1) contains three *BSTU*I restriction sites, yielding fragments of 208 bp, 57 bp, 54 bp and 44 bp.

Example 2: Results

Hypermethylation was detected in 4/43 (9%) primary glioblastomas. 11/13 (85%) secondary glioblastomas, 5/9 (56%) anaplastic astrocytomas, 5/7 (71%) low-grade astrocytomas, 6/6 (100%) OII, 4/5 (80%) AOIII, 3/3 (100%) AOAIII and 5 out of 7 (71%) glioma cell lines.

## Claims

**1.** A method for diagnosis or prognosis of a cancer which comprises determining the methylation degree of the *DUSP4* gene, preferably that of the dual-specificity phosphatase 4/map kinase phosphatase 2 *DUSP4* promoter in a DNA sample, wherein the methylation degree of selected CpG sites in the *DUSP4* gene/promoter is indicative for a cancer or cancer risk.

**2.** A method for prediction of the responsiveness of tumors to agents acting on the amplified/mutated EGFR gene which comprises determining the methylation degree of the *DUSP4* gene, preferably that of the dual-specificity phosphatase 4/map kinase phosphatase 2 *DUSP4* promoter in a DNA sample, wherein the methylation degree of selected CpG sites in the *DUSP4* gene/promoter is indicative for the responsiveness of the tumor.

**3.** A method for determination of the methylation degree of the dual-specificity phosphatase 4/map kinase phosphatase 2 (*DUSP4)* gene 5'non-coding region ("*DUSP4* promoter") in a DNA sample, which comprises

(a) treating the DNA sample with bisulfite;
(b) amplifying a segment ("amplified segment") of the bisulfite-modified DNA ("bisulfite-treated counterpart") obtained in step (a) by PCR using the bisufite treated DNA as template, wherein the amplified segment comprises at least 20 consecutive bisulfite-modified nucleotides within a 5' untranslated region of the native *DUSP4* sequence represented by bp 1 to 8276 of SEQ ID NO:1 and spans at least three CpG sites ("selected CpG sites"); and
(c) analysing the amplification product and determining the methylation status of the selected CpG sites in the amplified segment.

**4.** The method of claim 3, wherein

(i) the amplified segment in step (b) spans at least 4 selected CpG sites, preferably the at least three(four) selected CpG sites covered by the amplified segment are selected from the group consisting of the CpG sites at positions nt 709 + 710 (CpG 1), nt 715 + 716 (CpG 2), nt 722 + 723 (CpG 3), nt 724 + 725 (CpG 4), nt 730 + 731 (CpG 5), nt 733 + 734 (CpG 6), nt 742 + 743 (CpG 7), nt 779 + 780 (CpG 8), nt 781 + 782 (CpG 9), nt 786 + 787 (CpG 10), nt 790 + 791 (CpG 11), nt 801 + 802 (CpG 12), nt 818 + 819 (CpG 13), nt 823 + 824 (CpG 14), nt 825 + 826 (CpG 15), nt 838 + 839 (CpG 16), nt 912 + 913 (CpG 17), nt 915 + 916 (CpG 18), nt 926 + 927 (CpG 19), nt 946 + 947 (CpG 20), nt 949 + 950 (CpG 21), nt 977 + 978 (CpG 20), nt 1005 + 1006 (CpG 23) in the native *DUSP4* DNA sequence represented by SEQ ID NO:1, and/or

(ii) the amplified segment comprises at least 20 consecutive nucleotides of the bisulfite-treated counterpart of the sequence represented by nt 241 to 6129 of SEQ ID NO:1 and preferably comprises the bisulfite-treated counterpart of the sequence represented by nt 681 to 1032 or 1392 to 1890 of SEQ ID NO: 1; and/or

(iii) the primer pair used in the amplification step (b) consists of a forward and reverse primer, wherein the forward primer is complementary to at least 10 consecutive nucleotides of nt 681 to 705, nt 602 to 622, nt 473 to 493 of SEQ ID NO:1 or its bisulfite-treated counterpart, and the reverse primer is complementary to at least 10 consecutive nt of nt 1007 to 1032, nt 1065 to 1085, or nt 1136 to 1156 of SEQ ID NO:1 or its bisulfite-treated counterpart, and wherein preferably at least one of the primers is a primer complementary to said segments of SEQ ID NO:1 after the bisulfite modification.

**5.** The method of claim 4, wherein the at least three selected CpG sites covered by the amplified segment are selected from the group consisting of CpG 1 to 23, preferably of CpG 3 to 15, more preferably of CpG 3 to 9.

**6.** The method of claim 5, wherein the selected CpG sites covered by the amplified fragment comprise either (i) CpG 3 to CpG 4 or (ii) CpG 14 to 15.

**7.** The method of any one of claims 3 to 6, wherein step (c) comprises Pyrosequencing™ or restriction analysis of the amplification product.

**8.** The method of claim any one of claims 3 to 7, wherein
step (b) comprises only one PCR amplification reaction;
the analysis step (c) is performed using Pyrosequencing; and the selected CpG sites comprise at least three CpG sites selected from CpG 3 to 15, and more preferably comprise all CpG sites CpG 3 to 9.

**9.** The method of claim 8, wherein

(i) in the primer pair used in the amplification step (b) the forward primer is complementary to at least 10 consecutive nucleotides of nt 681 to 705 of SEQ ID NO:1 or its bisulfite-treated counterpart, and the reverse primer is complementary to at least 10 consecutive nt of nt 1007 to 1032 of SEQ ID NO:1 or its bisulfite-treated counterpart; and/or

(ii) in step (c) a segment comprising at least nt 706 to 1006 of SEQ ID NO: 1, or the reverse strand of said segment is pyrosequenced, preferably using a primer which is complementary to at least 10 nt downstream of said segment.

**10.** The method of any one of claims 3 to 7, wherein the analysis step (c) comprises a restriction digestion of the amplification product and subsequent analysis of the restriction products, preferably BstUI digestion; and the selected CpG sites comprise at least two pairs of CpG 3 and 4, CpG 8 and 9 and CpG 14 and 15, preferably comprise CpG 3, 4, 8, 9, 14 and 15.

**11.** The method of claim 10, wherein

(i) step (b) comprises two PCR reactions, a first PCR reaction with a first primer pair using the template as defined in claim 1, and a second PCR reaction with a second primer pair using the amplification product of the first PCR reaction and a second primer pair, wherein the first primer pair contains 5'-tails of at least 10 nt length which are identical to the second primer sequences, and in the second primer pair one primer is labelled at its 5'tail with one dye molecule and the second primer is labelled at its 5'tail with a different dye molecule; and

(ii) step (c) is performed on the amplification product of the second PCR reaction.

**12.** The method of claim 10 or 11, wherein

(i) in the primer pair used in the first PCR in amplification step (b) the forward primer is complementary to at least 10 consecutive nucleotides of nt 681 to 705 of SEQ ID NO:1 or its bisulfite-treated counterpart, and the reverse primer is complementary to at least 10 consecutive nt of nt 1007 to 1032 of SEQ ID NO:1 or its bisulfite-treated counterpart;
(ii) the primers of the primer pair used in the second PCR are at least 13 nt long and are labeled at their 5'ends with different fluorescent or luminescent dye molecules;
(iii) the amplification product which is analyzed in step (c) is labelled with two dye molecules which are different from each other, wherein one dye molecule is covalently linked to the 3'end and the other to the 5'end of the amplification product, and wherein preferably said amplification product is the result of the second PCR in step (b) using the labelled primers as defined in (i) above; and/or
(iv) at least one restriction endonuclease used in the restriction digestion of step (c) has a recognition site which spans at least one of the selected CpG sites and does recognize only a recognition site containing CpG at the CpG site in the amplification product obtained by step (b).

**13.** The method of any one of claims 3 to 12, wherein the DNA sample is

(i) genomic DNA; and/or
(ii) isolated from mammalian, preferably from human tissue, and wherein said tissue is preferably a kryopreserved or formalin-fixed tissue; and/or
(iii) isolated from a tumor tissue, preferably tumor tissue isolated from glioma, diffuse large B-cell lymphoma (DLBLC), colorectal carcinoma, non-small cell lung carcinoma, lymphoma, head and neck carcinoma, brain tumors, more preferably tumor tissue isolated from glioma, colorectal carcinoma and DLBLC, most preferably tumor tissue isolated from malignant glioma, especially glioblastoma, and DLBLC.

**14.** The method of claim 2, wherein

(i) the determination is performed by the method of any one of claims 3 to 13; and/or
(ii) a methylation degree of 75 % or more of the selected CpG sites indicates that the tumor is susceptible to agents acting on the the amplified/mutated EGFR gene; and/or
(iii) the method is suitable for the responsiveness of gliomas.

**15.** The method of claim 1, wherein

(i) the determination is performed by the method of any one of claims 3 to 13; and/or
(ii) the method is suitable for identifying secondary glioblastomas.

**16.** An isolated DNA molecule which is

(i) the amplification product as obtainable by step (b) according to any one of claims 3 to 13;
(ii) a primer for the PCR reaction in step (b) as defined in claim 2 (iii), preferably as defined in claims 9 (i), 11 (i), 12 (i) or 12 (ii); or
(iii) a primer for the pyrosequencing reaction as defined in claim 9 (ii).

**17.** A kit for performing the method of any one of claims 1 to 15, which comprises one or more of the primers as defined in claim 16 and/or one or more of the restriction endonucleases as defined in claim 12 (iv).

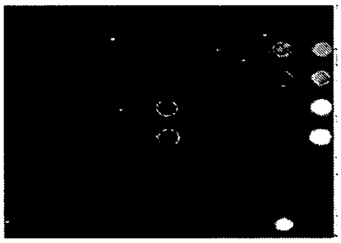


Fig.1a

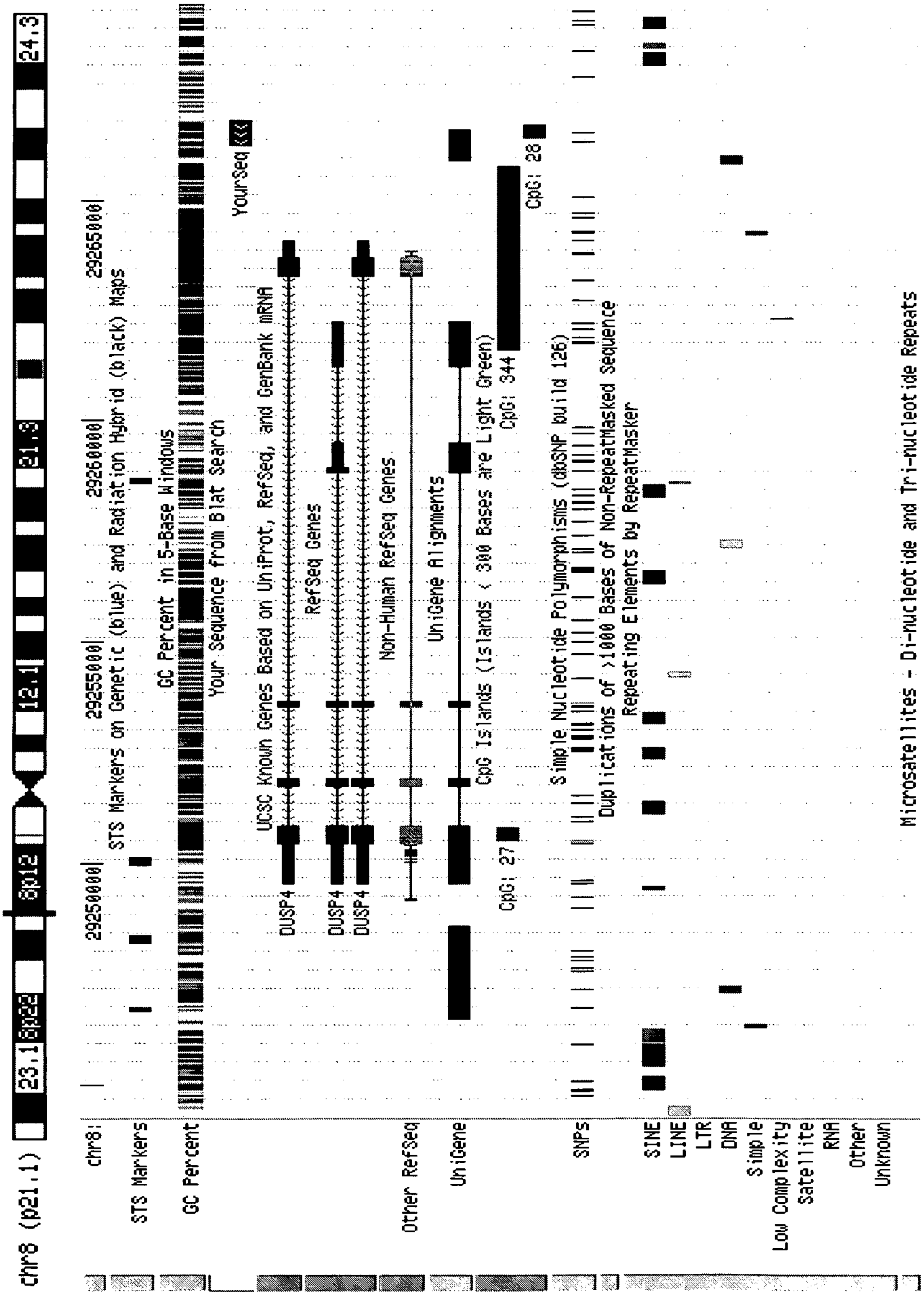
chr8 (p21.1)
23.1
8p22
8p12
12.1
21.3
24.3
chr8:
29250000|
29255000|
29260000|
29265000|
STS Markers on Genetic (blue) and Radiation Hybrid (black) Maps
STS Markers
GC Percent in 5-Base Windows
GC Percent
Your Sequence from Blat Search
YourSeq
UCSC Known Genes Based on UniProt, RefSeq, and GenBank mRNA
DUSP4
RefSeq Genes
DUSP4
DUSP4
Non-Human RefSeq Genes
Other RefSeq
UniGene Alignments
UniGene
CpG Islands (Islands < 300 Bases are Light Green)
CpG: 27
CpG: 344
CpG: 28
Simple Nucleotide Polymorphisms (dbSNP build 126)
SNPs
Duplications of >1000 Bases of Non-RepeatMasked Sequence
Repeating Elements by RepeatMasker
SINE
LINE
LTR
DNA
Simple
Low Complexity
Satellite
RNA
Other
Unknown
Microsatellites - Di-nucleotide and Tri-nucleotide Repeats

Fig.1b

**CG**ctttt**CG**caattcaagtggctct**CG**gcttaaacacattaagtcccaagctgtgactcccttccagtgaa
cagc**CGCG**tagg**CGCG**ggca**CG**caca**CG**tggacacacacactcaca**CGCG**ccctcccacacccctttc**CG**
tgtgaat**CG**cctg**CG**ggatttagggcttctccaccag**CG**attactgtcaggtgcagaaagcaagcctctt
tgttctatgggcattttcctccccctggatttgactgttatttactggcca**CG**tgag**CG**ccagg**CGCG**gg
tc**CG**g**CG**gggaagg**CG**accccagaacagggtagacaaaagctggcccagag**CGCG**acc**CG**tg**CG**ccccag
gga**CG**tctccccagctcctg**CG**aca**CGCG**tgaaattggcc**CG**gacctgcctcctgagaggcttttcccag
aaatgtcttgttaaagcctttgtaaaggaatcagtgtttacttg**CG**c**CG**gagagctc**CG**gccctgggtc
ctggcagg**CG**g**CG**gatttctgagctgctggagagcaacc**CG**ggaaagcaaaatcagccttggactgt**CG**

**Fig.1c**

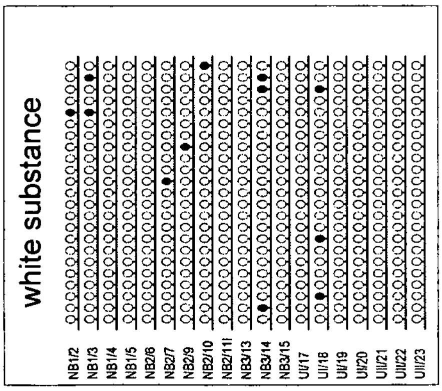


Fig.2a

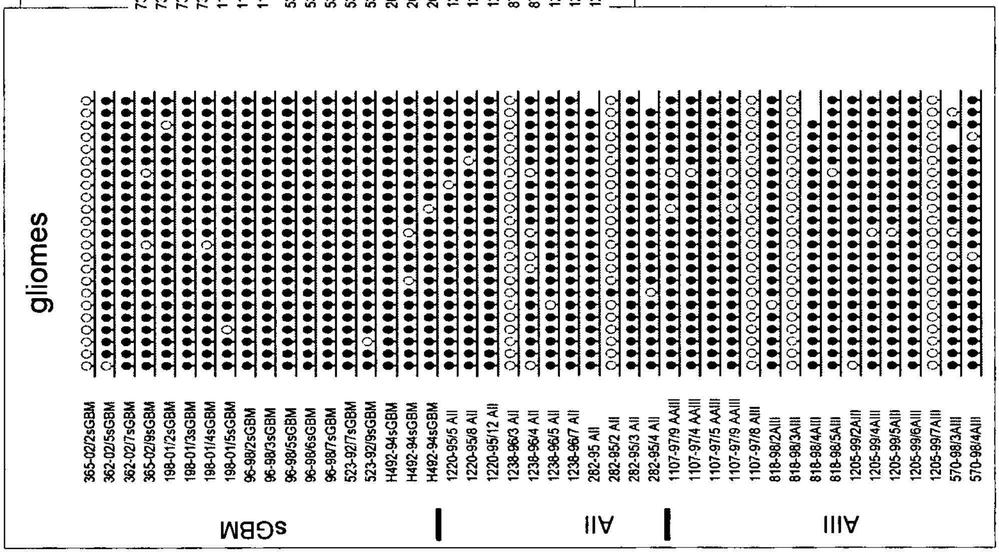


Fig.2b

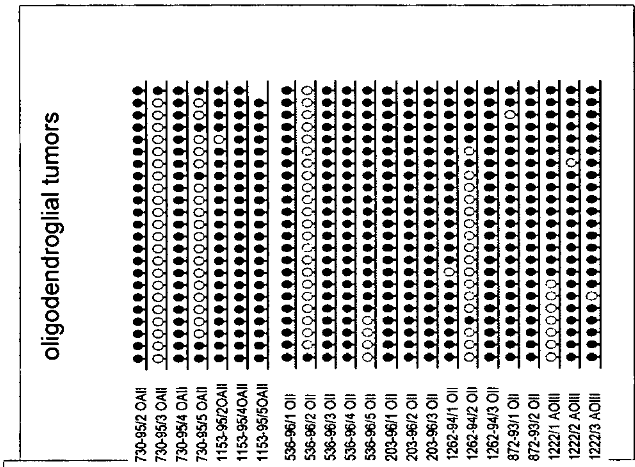


Fig.2c

Fig.3a

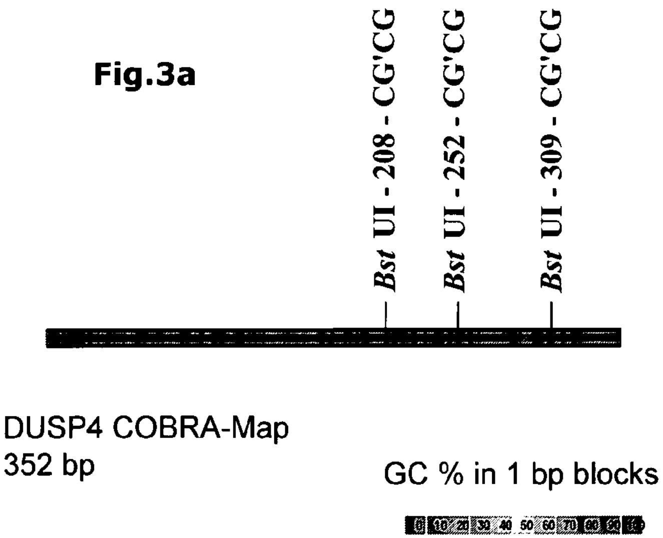


Fig.3b

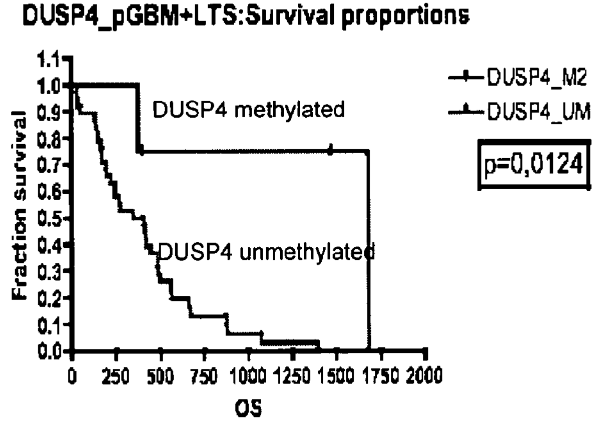


Fig.3c

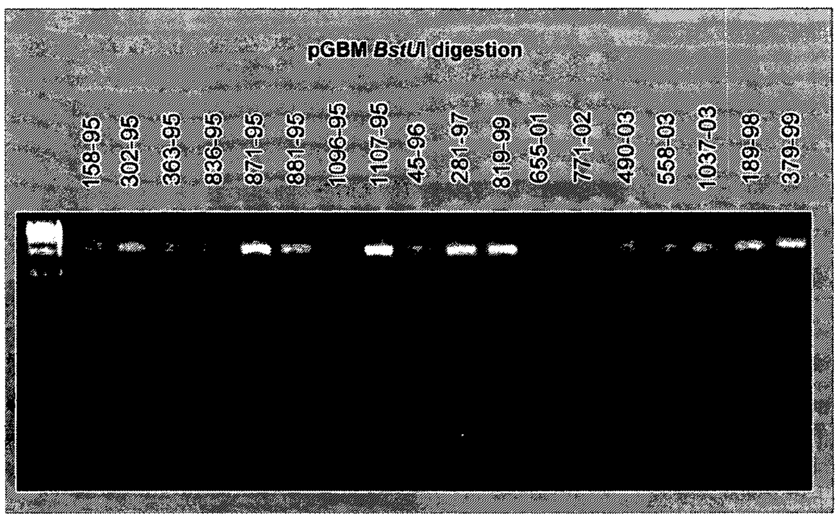


Fig.3d

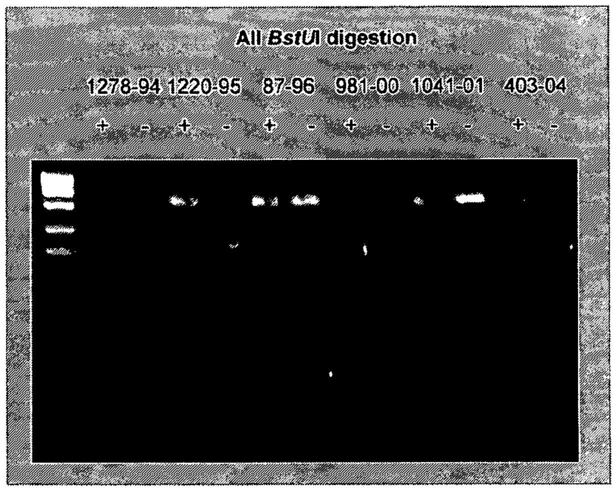

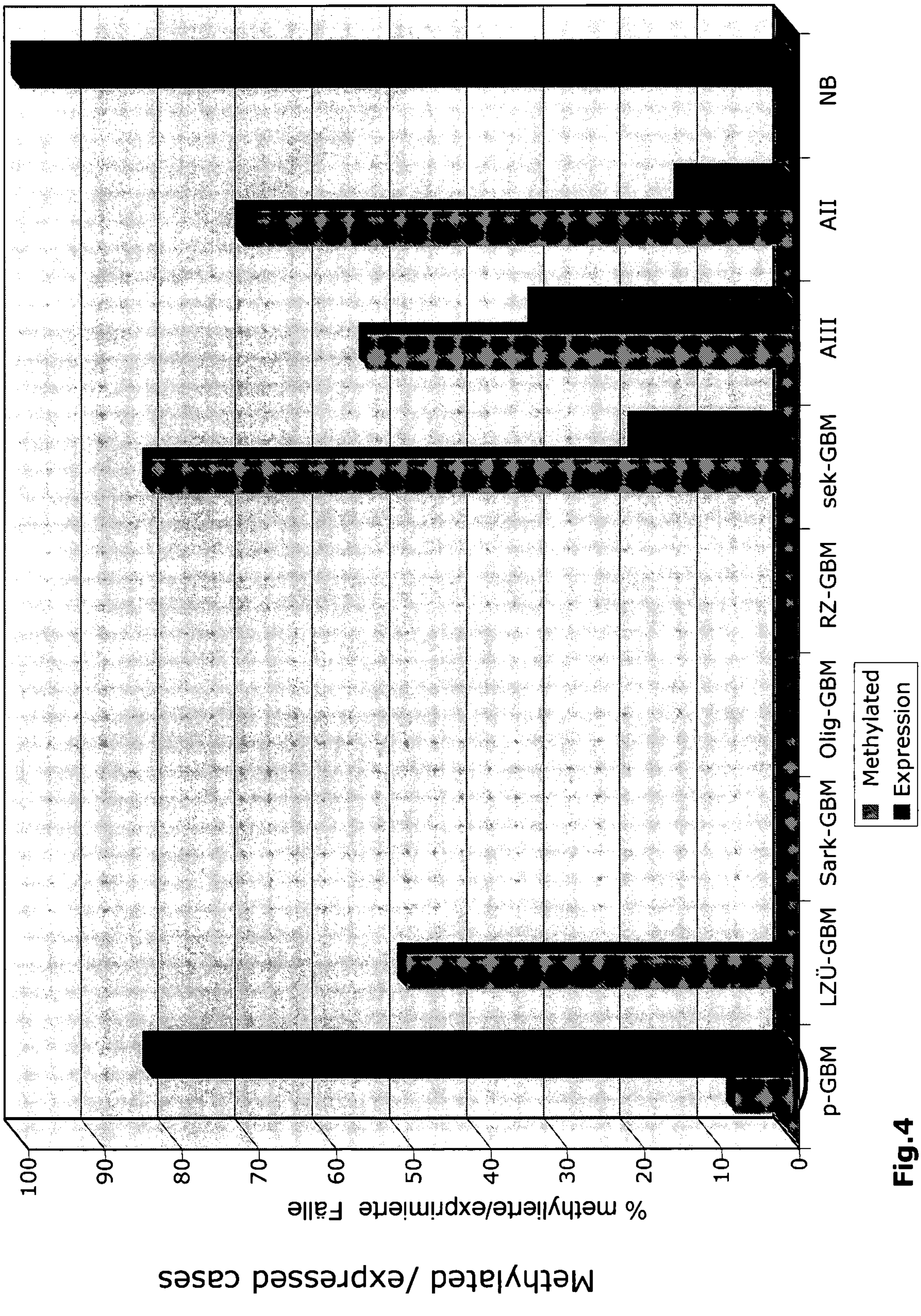
100
90
80
70
60
50
40
30
20
10
0
% methylierte/exprimierte Fälle
Methylated /expressed cases
p-GBM
LZÜ-GBM
Sark-GBM
Olig-GBM
RZ-GBM
sek-GBM
AIII
AII
NB
Methylated
Expression

**Fig.4**

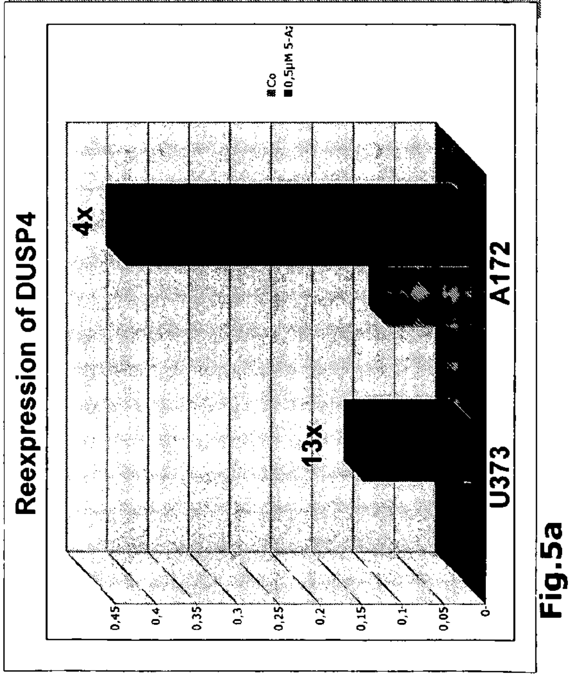

Fig.5a

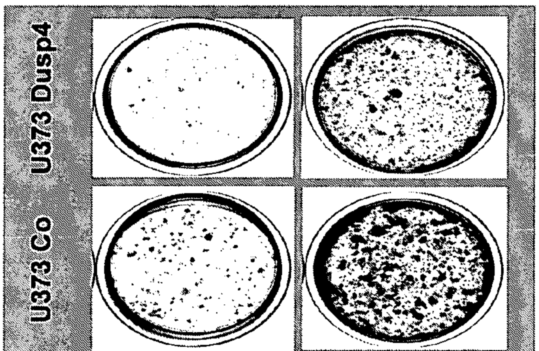

Fig.5b

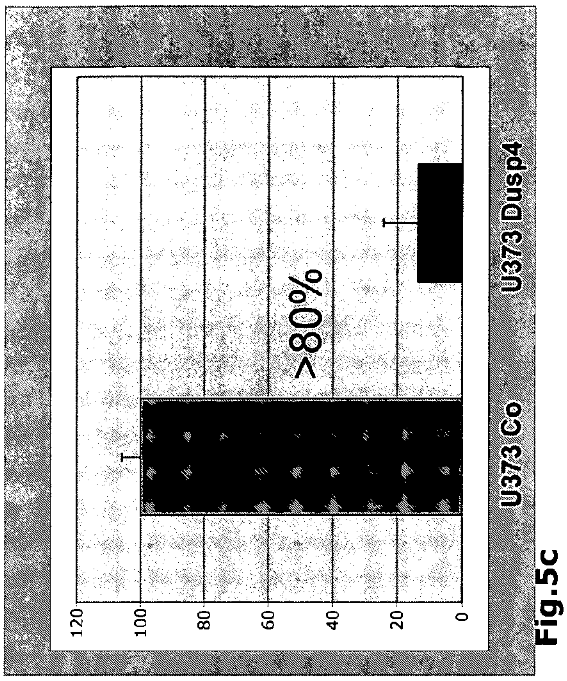

Fig.5c

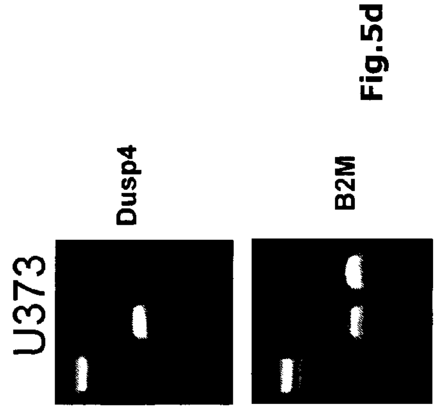

Fig.5d

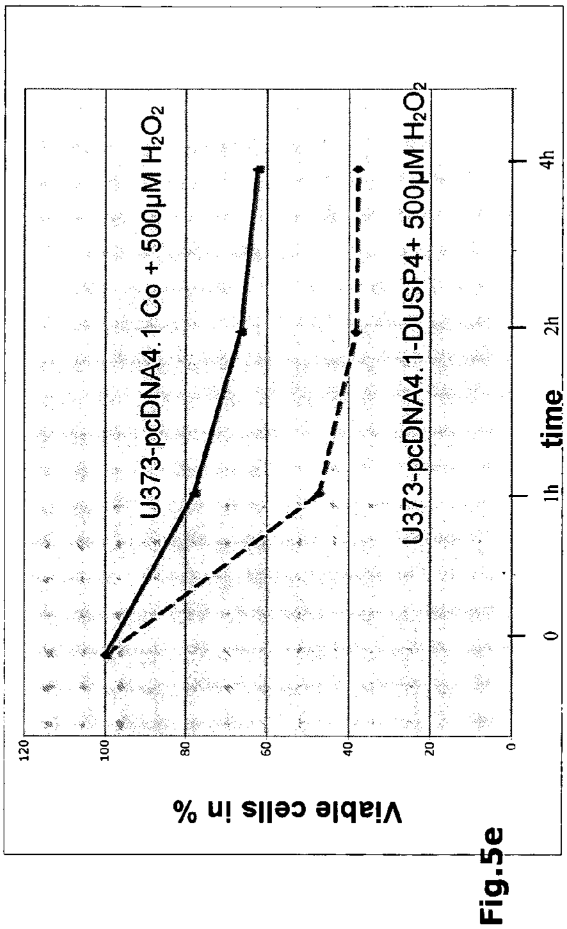

Fig.5e

## Fig.6a

atataaataaaagacctctccttgggcaatcccatcccaattcctacaaatatgcctt
agttaattaaattaactttgaaatgacattc**CG**tgtcttgctccaaattgcctttgtt
ttctccaagatctgctcattgacaagctgtttcacaaggttcctgtttaaaatggtta
tgttt**CG**tccacagactgcaga**CG**aattggagacaggttttatctatagc**CG**gcatgg
agtagccct**CGCG**ttgtactcatttaactgtggaccaatggttcacaaacatacaatt
aaaaaatatatatctagggtgtttacacagtatcc**CG**ataaatacaatatggtaactt
aaatctcctttgctgaaattcacacaactgaaggccaattcctgagttggcaatgggg
tgtggattttaaaaattagtctttttaaaaatcaggcctt**CG**cttt**CG**caattcaag
tggctct**CG**gcttaaacacattaagtcccaagctgtgactcccttccagtgaacagc**C**
**GCG**tagg**CGCG**ggca**CG**caca**CG**tggacacacacactcaca**CGCG**ccctcccacaccc
ctttc**CG**tgtgaat**CG**cctg**CG**ggatttagggcttctccaccag**CG**attactgtcagg
tgcagaaagcaagcctctttgttctatgggcatttcctccccctggatttgactgt
tatttactggcca**CG(1)**tgag**CG(2)**ccagg**CG(3)CG(4)**ggtc**CG(5)**g**CG(6)**
gggaagg**CG(7)**accccagaacagggtagacaaaagctggcccagag**CG(8)CG(9)**a
cc**CG(10)**tg**CG(11)**ccccaggga**CG(12)**tctccccagctcctg**CG(13)**aca**CG(**
**14)CG(15)**tgaaattggcc**CG(16)**gacctgcctcctgagaggcttttcccagaaat
gtcttgttaaagcctttgtaaaggaatcagtgtttacttg**CG(17)**c**CG(18)**ggaga
gctc**CG(19)**gccctgggtcctggcagg**CG(20)**g**CG(21)**gatttctgagctgctgg
agagcaacc**CG(22)**ggaaagcaaaatcagccttggactgt**CG(23)**gggcccctgg
ggctccttgctccttat**CG**aaag**CG**agttattagatagacccccacagtcccattca
gcagaatgggaaactggactcagacacctttgccctccccacagggaggcca**CG**c**CG**
tg**CG**gctttgaccactgatctcccacc**CG**gagtgcc**CG**a**CG**cccagggtcctgccac**C**
**G**ggcagcct**CG**gtcagtccacagttgtggctccttccagggcctggactagggtgagc
acaagccttgag**CG**caacatttaagaaggg**CG**c**CG**aaaagtcagtaatcaaaagaaat
atcttgatgcaatgcctctttaaaagaaaaaaaatgcaaaaaatccatgatgaataa
aatactaaattttaaaaagagaaaggatc**CG**tgcagtgccat**CG**taagccattttgga
gcc**CG**gagcaaaaggaaatatcaccttgcccaag**CG**gtgcccctacagctcatc**CG**ag
tagggcc**CG**gggt**CG**aggcatt**CG**ggcccagtgggga**CG**aggccagt**CG**aaggtc
t**CG**gaagtggaggctc**CG**c**CG**agctc**CG**gt**CG**ggagtgcagggatggcccc**CG**ggac
**CG**aggtgagagct**CG**ggaaagc**CG**ctc**CG**cc**CG**gaacaaaggcatggggagagggtg
agcttggg**CG**ggagagacc**CG**g**CG**ggtac**CG**gtgccct**CG**ctgcctgggt**CG**ggcttc
ca**CGCGCG**ccc**CG**gaatggaata**CG**ctactctgcagcctc**CG**aaactg**CG**ag**CG**agtc
ctgtaactcccttgctctgtgattaattctcactaacaagacttggcaagatgt**CG**gg
**CG**aatgattttggcttctgca**CG**gtccccac**CGCG**tg**CG**tgcacaaaccccagcca
aaagc**CG**cctctgggaaattaaatgcaaaagagaaatggggatggggagggctgcta**C**
**G**tgaccaggaaaaagggatgcccagaaacatgaat**CG**gacccagagctgctgaagtcc
tttcaaaaggtcattctttg**CG**ggtacattttccagggtccagctc**CG**caacaaatgt
ggaccctgtcatttcctgaaaggataattcacaactatgcaagatagggtgaaga**CG**t
ttcccaaacc**CG**aaaaccttgttttcccc**CG**accagggttaaataaacatcttttag
gaag**CG**tggacaggag**CG**cagcctgctctcctccct**CG**gaacaccattc**CG**gcaatta
atgcctccctttgggtagtaaagcaacaaaccccacacctcactc**CG**atcctgggctt
**CG**gg**CG**ggaggactttctctttcatcttccaagcagggggttgccca**CG**ttcttgggg
aagctataaaactgatttaatggctttagatgaaaat**CG**atca**CG**ctaatgcata**CG**c
taa**CG**tctcaggaat**CG**catattcagaaaggactggc**CG**ggc**CG**aaag**CG**ca**CG**ggga
gtctggggctaggaggtgtcaggccc**CG**ctgggtggcagcag**CG**ctc**CG**gtcccctc
tccacttgggtaac**CG**ggaaaaccta**CG**ggctgtca**CGCG**gggaag**CGCG**aaggtg

ccaagggatgaaagctcaaacc**CG**agccctggcctcctcagc**CG**gctatttcctttgg
**CG**c**CG**cc**CG**cctag**CG**g**CG**gggtgcag**CG**g**CG**gcacaggtgc**CG**gtgt**CG**ggctggag
g**CGCG**g**CG**caggctgggcc**CGCG**ggtaga**CG**g**CG**aaagg**CG**c**CGCGCG**ctccattcac
aaagtc**CG**gg**CG**ctgcc**CG**c**CG**ctgg**CG**g**CG**ggt**CG**gaggc**CG**cctccctcttcctct
**CG**gcct**CG**gttttatgaatgggcctgatgg**CG**agcacc**CG**g**CG**ccctgtttactc**CG**c
tctttgtga**CG**t**CG**agttcc**CG**tgac**CG**ggagccag**CG**gc**CGCG**ctccattcaagctc
**CG**ggagggggtgggaggaggggcc**CG**gaggggg**CG**gggagtcag**CGCG**ggggg**CG**gg
ggacag**CGCG**ggggg**CG**gggga**CG**g**CGCG**gggcc**CG**gaatggaa**CG**ggg**CG**gggcctg
g**CG**gggtagtacctag**CG**cccccctcccc**CG**ggag**CGCG**gaggagcattaataaacctc
taagc**CG**aggagaaactctggctggggcagtg**CG**ctgag**CG**c**CG**gaggag**CG**taggc
agggcag**CG**ctgg**CG**ccagtgg**CG**acaggagc**CGCGCG**ac**CG**gcaaaaataca**CG**gga
ggc**CG**t**CG**c**CG**aaaagagtc**CGCG**gtcctctct**CG**taaacacactctcctccac**CG**g**C**
**G**cctccccctc**CG**ctctg**CGCG**c**CG**cc**CG**gctggg**CG**cc**CG**aggc**CG**ctc**CG**actgct
atgtgac**CGCG**aggctg**CG**ggaggaaggggacagggaagaagaggctctcc**CGCG**gga
gcccttgaggaccaagtttg**CG**gccacttctgcagg**CG**tcccttcttagctct**CG**cc**C**
**G**cccctttctgcagcctagg**CG**gcc**CG**ggttctcttctcttcct**CGCGCG**cccagc**CG**
cct**CG**gttcc**CG**g**CG**accatggtga**CG**atggaggagctg**CG**ggagatggactgcagtg
tgctcaaaaggctgatgaac**CG**gga**CG**agaatgg**CG**g**CG**g**CGCG**gg**CG**gcag**CG**gcag
cca**CG**gcaccctggggctgc**CG**ag**CG**g**CG**gcaagtgcctgctgctggactgcagac**CG**
ttcctgg**CG**cacag**CGCG**ggctacatcctaggtt**CG**gtcaa**CG**tg**CG**ctgtaacacca
t**CG**tg**CG**g**CG**g**CG**ggctaagggctc**CG**tgagcctggagcagatcctgcc**CG**c**CG**agga
ggaggta**CGCG**cc**CG**cttg**CG**ctc**CG**gcctctact**CG**g**CG**gtcat**CG**tcta**CG**a**CG**ag
**CG**cagcc**CGCGCG**c**CG**agagcctc**CGCG**aggacagcac**CG**tgt**CG**ctggtggtgcagg
**CG**ctg**CG**c**CG**caa**CG**c**CG**ag**CG**cac**CG**acatctgcctgctcaaaggtaaa**CG**agggct
c**CG**gg**CG**ctagctggagt**CG**ggagatggaggggtcttgg**CG**gcctg**CG**ccttattg
gca**CG**gaaggagtccc**CG**g**CG**tgatt**CG**tggttg**CG**ggatccc**CGCGCG**cccttt
gtgtgtgttggtgtgtaggggtttgcaaggtttctgtgcaagtg**CG**aggttcaagtcc
tcagggaaagtg**CG**ga**CG**ggtttctc**CG**gtcacctgcaattc**CG**cac**CG**tcctag**CG**a
ggccc**CG**tttattgacattaa**CG**gtcctagcct**CG**aggatgaag**CG**actcctctg**CGC**
**G**gtccatcttgagtcc**CG**ggaacagtttccaagtacca**CG**ggg**CGCG**tggggacaaga
taggcctgttggtttgg**CG**tgctggggccctacactgagc**CG**gac**CG**ggagaatccca
gaggtggagaaaaggtgaagaggggtggg**CG**c**CG**aggtacaattggccttgccagttt
cactgaaaacaagaccctggcaaatgcagaaagggagattgggttgcacacactgtgt
agag**CG**atctgtatctg**CG**ttcc**CG**ctgcccaactccaagctcagcttggaaggtggc
caatccc**CG**tcc**CG**ggctagatccaggctctgggag**CG**ggaggctgaaggactgggta
tctgggga**CG**c**CG**gg**CG**gaac**CG**gtcctctag**CG**cctgcaaatc**CG**ggcag**CG**gaga
atgatcacctttgttcagcc**CG**gatttt**CG**actatctgatgg**CG**g**CGCG**tgc**CG**gat
tttag**CG**agccctgg**CG**aaactct**CG**g**CG**g**CG**agactgtcttgtcacatttggtagt**C**
**G**gaaatcctggaaaaat**CGCG**tgctgtc**CG**agtccctggagggacctgg**CG**cc**CG**cca
cacctgcagggcagggtgggtc**CGCG**gccct**CG**ggctagggag**CG**ggcaggg**CGCG**tg
gggtc**CG**gg**CG**ccccc**CG**c**CGCG**ctg**CG**cctc**CG**cctcc**CG**c**CG**c**CG**cattc**CGCG**ca
ttcttc**CGCGCG**ggtagggtctg**CG**cttc**CG**agcc**CG**gtagGCAGTTCAGACCCCCCC
ACACCCATCAAAGAGC**CG**CTCCTCCCCCC**CG**CAGG**CG**CCTT**CG**C**CG**CCTCCCTCCCTT
CCTTTCCTTTC**CG**CTCCTCTTC**CG**ACCTGTCCACC**CG**GGAGGAAGGGAGCTGGAAAGG
GGG**CG**GAAACCTCTCCCCTCCAAAAAGCACAACAAAACTGTTCAGTG**CG**GAGGAGC**CG**
GGTT**CG**CCCCTGC**CG**GACAG**CG**GGGGGCTTTGTTCCC**CG**CAGTTGTTTCCTGCCCATT

TGACCTGTCAGCTGCTGGGGAAA**CG**CTGCTGTTGACCTTTGGTTGAACTGCTAAGG**CG**
ATTTTGCTGATTTTTCTTTCTTTTTC**CGCG**AGGGCTGTCTTTTGCTCCTCCAAATGAG
CCCAGTCCCCCTCCCTTCTCCCCAAAG**CG**CTCCAAGAGAAAGTGCCAGGAAGGGGCTT
GTCC**CG**GAAGGCCTGG**CG**GCTGAG**CG**GGGCCAGGTCCTGGTTAGGCCACCAGGGTGGG
**CG**TC**CGCG**CCATTGTTTGAGCTTGT**CG**G**CG**CTGGTGGGAGAGATGAGGGCAATTCCTC
TGGGA**CG**CAAGTCCCCT**CG**AATGGC**CG**GGGCTGGC**CG**GGATGTTCCC**CG**CA**CG**G**CG**CT
GCCCT**CG**AGTCCCCC**CG**ATGGAGAG**CGCG**GG**CGCG**CCTTCCTT**CG**CTGG**CG**TCCAAAC
C**CG**GGACCAGCTAGAACACAGCAGGGCTGGGACTGGGTTCCAGCCCCA**CG**TGGAGTCT
GGATTTGTTTTGTTGTGTTTTGCTTTCCTTCCTGGAAGAAATCC**CG**AGGGGAC**CG**CCC
TAGAG**CG**GCAGCTCCAGGACCT**CG**GCCCTTGGGCTTC**CG**GGGGTGCAGCCACTTAGGC
CC**CG**CTCC**CG**GGAGAGAGGGATTATTTTTAAGATTTATCCCCAGGG**CGCGCG**GCAT
TTCCCTGTCCCT**CG**TGAATCC**CG**TTGAGAGTCCTCCCTCCCCAACCTCCTCCATTTCC
CCAGCCAGAC**CG**ATT**CG**AGAGCCCTGGAGATTCTGGG**CG**AGGCTAGTGACTGGGTAGT
ACAGGCCTCTAGgtaaaatgacagccctctggtgggtgg**CG**aggtgcctggccaacat
aagagaagtactttaagggctgtttcctcctggagcctgcactgcctct**CG**tagt**CG**g
ccc**CG**a**CGCG**ggcagggagaatcatcttctgcatctcaaagtggacatccttgcagat
ggaggtgtgcaggcctgggccacagatttagctt**CG**gtctcttctagaatagcccctc
ctagccagaagttcagcttctgcttccaaagggagcctgcccttggaagcaagcacct
aaaattgagaggtgggggaatcttccagcctgctctgttgtggtgaacat**CG**ttgagg
ttaactgggccctatt**CG**gtttagcttagctgatttgacatatgtgttcagcagagg
ccatctttaacccagttcctcttcttcctctttgccctctaatggaaaggcatcccta
gagaggtcagaaggaaaaagtcagtatctgctcccacagctctg**CG**ggaaggggctgg
ggaaggaaatcaaggtgtctcccaggtctccagatacacctcccttccatgcctggaa
gccagtgcagtggagcaaatgtggtttcc**CG**ggagggggctctggggcagagacctaa
aggggaattttgagaagcaaaggaagaaaaagccccttagattatctttagtaaatgc
ctgaattggtgaagggttggagaatttgtttcctactggagtaattcattttatttc
cag**CG**agggaatgtatgtatgtctcatttcaggtgtgcctgtg**CG**tgtctgtaaagca
ggacaggaagccagtggaaggcttgatggttggtgtctgccattcttggaggggttag
gagaagcagtaaaagcaacaagagtattttcagaagtgatagtagcatgtccagccaa
agctatctaattctattaattgcattttaaaattcagtttttctatttctgcca**CG**tt
gaagggttacttcaacca**CG**ttgaactggttactccatgcaggtggcaggaggtaaac
tt**CG**cttaatatcaagaaagtacagcctagcctttacatttcctcagattacc**CG**act
tttgctaaaagcaataa**CG**aaaaatctaatgttttctatctttgctagtaaaggattt
ccagtggggtaatttttttaaaactcatttaatttaaaatcttgtaaggcatttttag
agtcagtttggaaattttagtttttcagatttatcacaatacacttcttgaaatccc
tttctctctcatt**CG**taaatccttggattagagtttgagactcccctggtcattagag
aaagacaaaaagagcttttcttaccctattctttgcctttattaaccccaaataaca
aaataatctgttatgcaaaaagattgcaggtataaaaacattaccttcctttccacct
acctaaaacttccctcagcctttgtcagttctaatttaaaagctgtcctggagctgtt
ggagaaatggggaagatcatctagatattccagtttctccttccactcaccctatagt
ac**CG**gatagcatccaatagcccagggcctgaccatacagcccatgatattaccctttc
tgtagaaaacagtcactaaggccactgatgaatgtgggggagaggaacacaattaac
agctctggaaactctggtgacattttctgtttttctctctctagCCCCACCATTGC
TCTCTCTGTCTTCAGTTCCCCAGGAGGGCAATGGCATCAAACAGCACAGCTCTGGGGG
ATGTCAATATTGCATACCTTTTCTACCTAAAGGGAAAATGACT**CG**CTTTTCTGCTTGC
AAATATGGTAGTTTCTGCTTACAAATGTAATACAATGCCCATGACAGCCAAGGACTGG

AAGCATAAGTTGCTAGGTCTTACAGGTGATTTTTTACAATGAAGCAAACTCACTATGT
TAGACACCATTTACATTGGATGTCTCCAACTAACAAAAGTAACTAAAGACAGATGTAG
GTGTAAATTGAGAGTGAAATTTGACCCTTTAGAC**CG**TCACAACTTCCTTGGGCTTATC
CTGGGTGCTTATAGGAGAGGTGGGCTCCACCCACAAAAATGGACTGCTCAGAAAAATG
AGGGAGAGAGAAAGGGTGGCCACTTTCC**CG**AGCCAAGAAATTCCTTGAAAAAAAATCA
GAACATCTGAAACCAGAGAGC**CG**ATTTCCTTAC**CG**GGAGGCAGTTCCTGGCTAA**CG**AA
GAGGAAGCA**CG**ATGGGAAGAAAAGTTCACTCCAA**CG**GAAGCCAGTTTGCTGAACATA
GCAGAT**CG**CCCAGGAGGACTGGGAGAGACTGCAAACCAGTT**CG**AGCCCCCAGCATGG**C**
**G**TTAGGTGTCAGCCAGCTGGCAGGAAGGTCCAGGTGTCTGTGTTCAGAGTCTCAAGgt
gagaagttcacttagttggattttaaatacagctcagcatttacataggtgcttacta
agtcaacagtcacataaagtagagttcccaaaggcaaatggccttcaaacaaccccag
actttcttgcaagagcatgacttttctggcaagagcaaggccatgagccctgcctggg
acctggcactattctgagtgttgggggcataatggggaaaaagtggcagccacattaa
gattcatgaccaaggc**CG**ggtgtggtggctcacacctgcaatcctagcactttgagag
actgaggcaggcagatcacttgaggccaggacaacatggcaaacttggtctctacaaa
aaatgcaaaaattagc**CG**gg**CG**agatggcatgtgcctgtaatctcagctactcaggag
gctgaagcaaggagattgcttgagcccagggaagtcaaggctacagtgagccataat**C**
**G**tgccactgcactgcagcctgggtgagagagtgacagtgacagaccctgtctggggaa
aaaaaaaaaaaaaagatccatgaccatagtcaccaccaaaggacagtcttccccatca
caaatcccagtaacagtcctattatggtgata

## Fig. 6b

ggctaggacCGttaatgtcaataaaCGgggcctCGctaggaCGgtgCGgaattgcaggtgacCGgagaaaccCGt
cCGcactttccctgaggacttgaacctCGcacttgcacagaaaccttgcaaacccctacacaccaacacacacaa
aggggCGCGCGgggatcccCGcaaccaCGaatcaCGcCGgggactccttccCGtgccaataaggCGcaggcCGcc
aagacccctccatctcccCGactccagctagCGccCGgagccctCGtttacctttgagcaggcagatgtCGgtg
CGctCGgCGttgCGgCGcagCGcctgcaccaccagCGacaCGgtgctgtcctCGCGgaggctctCGgCGCGCGgg
ctgCGctCGtCGtagaCGatgacCGcCGagtagaggcCGgagCGcaagCGggCGCGtacctcctcctCGgCGggc
aggatctgctccaggctcaCGgagcccttagccCGcCGcCGcaCGatggtgttacagCGcaCGttgacCGaacct
aggatgtagccCGCGctgtgCGccaggaaCGgtctgcagtccagcagcaggcacttgcCGcCGctCGgcagcccc
agggtgcCGtggctgcCGctgcCGccCGCGcCGcCGccattctCGtccCGgttcatcagccttttgagcacactg
cagtccatctccCGcagctcctccatCGtcaccatggtCGcCGggaacCGaggCGgctgggCGCGCGaggaagag
aagagaaccCGggcCGcctaggctgcagaaaggggCGggCGagagctaagaagggaCGcctgcagaagtggcCGc
aaacttggtcctcaagggctccCGCGggagagcctcttcttccctgtccccttcctccCGcagcctCGCGgtcac
atagcagtCGgagCGgcctCGggCGcccagcCGggCGgCGCGcagagCGgaggggaggCGcCGgtggaggagag
tgtgtttaCGagagaggacCGCGgactcttttCGgCGaCGgcctccCGtgtattttgcCGgtCGCGCGgctcct
gtCGccactggCGccagCGctgccctgcctaCGctcctcCGgCGctcagCGcactgccccagccagagttttctc
ctCGgcttagaggtttattaatgctcctcCGCGctccCGgggggaggggCGctaggtactacccCGccaggcccC
GcccCGttccattcCGggcccCGCGcCGtccccCGcccccCGCGctgtccccCGcccccCGCGctgactcccCGc
cccctcCGggcccctcctcccacccctcccCGgagcttgaatggagCGCGgcCGctggctccCGgtcaCGggaa
ctCGaCGtcacaaagagCGgagtaaacagggCGcCGggtgctCGccatcaggcccattcataaaacCGaggcCGa
gaggaagagggaggCGgcctcCGaccCGcCGccagCGgCGggcagCGccCGgactttgtgaatggagCGCGCGgC
GcctttCGcCGtctaccCGCGggcccagcctgCGcCGCGcctccagccCGacacCGgcacctgtgcCGcCGctgc
acccCGcCGctaggCGggCGgCGccaaaggaaatagcCGgctgaggaggccagggctCGggtttgagctttcatc
ccttggcaccttCGCGcttcccCGCGtgacagcccCGtaggttttccCGgttacccaagtggagaggggacCGg
agCGctgctgcccacccagCGgggcctgacacctcctagccccagactcccCGtgCGctttCGgccCGgccagtc
ctttctgaatatgCGattcctgagaCGttagCGtatgcattagCGtgatCGattttcatctaaagccattaaatc
agttttatagcttccccaagaaCGtgggcaacccctgcttggaagatgaaagagaaagtcctccCGccCGaagc
ccaggatCGgagtgaggtgtggggtttgttgctttactacccaaagggaggcattaattgcCGgaatggtgttcC
GagggaggagagcaggctgCGctcctgtccaCGcttcctaaaagatgtttatttaaccctggtCGgggaaaaac
aaggttttCGggtttgggaaaCGtcttcaccctatcttgcatagttgtgaattatcctttcaggaaatgacaggg
tccacatttgttgCGgagctggaccctggaaaatgtaccCGcaaagaatgaccttttgaaaggacttcagcagct
ctgggtcCGattcatgtttctgggcatcccttttttcctggtcaCGtagcagccctccccatccccatttctcttt
tgcatttaatttcccagaggCGgcttttggctggggtttgtgcaCGcaCGCGgtggggacCGtgcagaagccaa
aaatcattCGccCGacatcttgccaagtcttgttagtgagaattaatcacagagcaagggagttacaggactCGc
tCGcagtttCGgaggctgcagagtagCGtattccattcCGgggCGCGCGtggaagccCGacccaggcagCGaggg
cacCGgtaccCGcCGggtctctccCGcccaagctcaccctctccccatgcctttgttcCGggCGgagCGgctttc
cCGagctctcacctcCGgtccCGggggccatccctgcactcccCGacCGgagctCGgCGgagcctccacttcCGa
gaccttCGactggcctCGtcccccactgggcccCGaatgcctCGaccccCGggccctactCGgatgagctgtagg
ggcacCGcttgggcaaggtgatatttcctttgctcCGggctccaaaatggcttaCGatggcactgcaCGgatcc
tttctcttttaaatttagtattttattcatcatggatttttgcattttttttcttttaaagaggcattgca
tcaagatatttctttgattactgacttttCGgCGcccttcttaaatgttgCGctcaaggcttgtgctcacccta
gtccaggccctggaaggagccacaactgtggactgacCGaggctgccCGgtggcaggaccctgggCGtCGggcac
tcCGggtgggagatcagtggtcaaagcCGcaCGgCGtggcctccctgtggggaggcaaaaggtgtctgagtcca
gtttcccattctgctgaatgggactgtgggggtctatctaataactCGctttCGaTAAGGAGCAAGGAGCCCCAG
GGGCCcCGacagtccaaggctgattttgctttccCGggttgctctccagcagctcagaaatcCGcCGcctgccag
gacccagggcCGgagctctccCGgCGcaagtaaacactgattcctttacaaaggctttaacaagacatttctggg
aaaagcctctcaggaggcaggtcCGggccaatttcaCGCGtgtCGcaggagctggggagaCGtccctggggCGca
CGggtCGCGctctgggccagcttttgtctaccctgttctggggtCGccttcccCGcCGgaccCGCGcctggCGct
caCGtggCCAGTAAATAACAGTCAAATCCAGGgggaggaaaatgcccatagaacaaagaggcttgctttctgcac
ctgacagtaatCGctggtggagaagccctaaatccCGcaggCGattcacaCGgaaaggggtgtgggagggCGCGt
gtgagtgtgtgtgtccaCGtgtgCGtgccCGCGcctaCGCGgctgttcactggaagggagtcacagcttgggact
taatgtgtttaagcCGagagccacttgaattgCGaaagCGaaggcctgatttttaaaaaagactaatttttaaaa
tccacaccccattgccaactcaggaattggccttcagttgtgtgaatttcagcaaaggagatttaagttaccata
ttgtatttatCGggatactgtgtaaacaccctagatatatatttttttaattgtatgtttgtgaaccattggtcca
cagttaaatgagtacaaCGCGagggctactccatgcCGgctatagataaaacctgtctccaattCGtctgcagtc
tgtggaCGaaacataaccatttaaacaggaaccttgtgaaacagcttgtcaatgagcagatcttggagaaaaca
aaggcaatttggagcaagacaCGgaatgtcatttcaaagttaatttaattaactaaggcatatttgtaggaattg
ggatgggattgcccaaggagaggtcttttatttatattcagcagtaaacctCGgggtgaaagaactttaaaaat

t**CG**ctgtgacaagtaaatccagcttctctttcatcagtcagtccttagac**CG**accttattagtcactgtcaggct
ccagcaaaacacctgctcttgtttaggaacatttctttccaattaggaagttgtatgat**CG**ggaatctaaagcc
**CG**gagtagaatccccagagagaagtacagcttctattctagttttgatgccaacctctgactcactgctctgtcc
caaagtgcctgaataaaacttgcctgaaaaatcattaatgaattaattaaggtggtgatggaaagaaaagtcctg
tggcttttcctttcttgagcaaagaggcagccttgctatta**CG**t**CG**aactggaacaggaataatccattttgcac
agatcttgttggttaggcttcttagatcaactaattagggaacttgccccctggtagtgggaaggctcaaaagaa
ctgggagaccctctcctgctcctgaaagcagagaagcctgtggtctacacag**CG**tgttcttaggcaa**CG**tttaga
aatggaattgagaaatgcctgtccttccta**CG**tacctctgcccatcaagtcaggactgaaag**CG**aaggaaagtc
ttcaaagttcacactgaacctaaccaaaaactctcaaagacatagcctgaggatttctttttttaagtcctgact
ttgttacagatctttttgttttctatgcttgatactctagcacttattaatgatttaaaggctatgtat**CG**gc
cagg**CG**aggtggctca**CG**ctagtaatcccagcactttggaagac**CG**aagtgggcagatcacctgaggtcaggagt
ttaagaccagcctggtcaacatggtgaaaccctgtctcttctaaaaatacaaaattagccagcatga**CG**gg**CGCG**
cacccataatcccagctacactggaggctgaggcaggagaat**CG**cttgaacc**CG**gaggtagaggttgcagtgag
ctgagatcccctcactgcactccagcctggg**CG**acaagagcaaaactccatctcaaaataaataaataaatacaa
ataaaaaataataaaagattaagtttctaacccagggtctctgcagaaaggttttaatgcattgctttatttact
gctttatttaaaactattcaatctaggctatgccaccatctagcttggtggccttgggcaagacagtctccttt
ggcctcactttcttcacctgcaaaatgaagagtttgggcaggatggtttctcaggtctggtctatttc**CG**accct
gtatgattctgagcagaagaagacttctcagctc**CG**agtccaagtttcataggtttcccatct**CG**ccctctgaag
agccaagcacactataatgccataagataaagga**CG**atctctagctcctccttgtccctgctttagaatataaac
tcatggacagcagagctcatagttttatttatttgtttagtca**CG**tgctaa**CG**tcccaagcatttttgataaaa**C**
**G**tcttcaaaatttcagaaacaaattttggaaagaaacacttctccaaa**CG**gataaaaatgaact**CG**gaggctgg
gcatggatggtggctcatgcctgtaatcccccagcactttgggaggctgg**CG**gatcacttgaggtcaggagttgg
agaccagcctgaccaacattaaaaatacaaaaattagtcaggcatgatggtgggtgcctgtagtcccagctact**C**
**G**tgaggctgaggcaaaagaatcccttgacc**CG**gagaggcagaggttgcagtgagtcaagattgggccactgcact
cctgcctggacaggg**CG**agacag**CG**

## Fig.6c

ggttaggat**TG**ttaatgttaataaa**TG**ggttt**TG**ttagga**TG**gtg**TG**gaattgtaggtgat**TG**gagaaatt**TG**t
t**TG**tatttttttgaggatttgaattt**TG**tatttgtatagaattttgtaaattttatatattaatatatataa
agggg**TGTGTG**ggattt**TG**taatta**TG**aatta**TG**t**TG**ggatttttttt**TG**tgttaataagg**TG**taggt**TG**tt
aagatttttttatttttt**TG**atttagttag**TG**tt**TG**gagtttt**TG**tttattttgagtaggtagatgt**TG**gtg
**TG**tt**TG**g**TG**ttg**TG**g**TG**tag**TG**tttgtattattag**TG**ata**TG**gtgttgtttt**TGTG**gaggtttt**TG**g**TGTGTG**gg
ttg**TG**tt**TG**t**TG**taga**TG**atgat**TG**t**TG**agtagaggt**TG**gag**TG**taag**TG**gg**TGTG**tatttttttt**TG**g**TG**ggt
aggatttgttttaggttta**TG**gagttttagtt**TG**t**TG**t**TG**ta**TG**atggtgttatag**TG**ta**TG**ttgat**TG**aattt
aggatgtagtt**TGTG**ttgtg**TG**ttaggaa**TG**gtttgtagtttagtagtaggtatttgt**TG**t**TG**tt**TG**gtagtttt
agggtgt**TG**tggttgt**TG**ttgt**TG**tt**TGTG**t**TG**t**TG**ttatttt**TG**ttt**TG**tttattagtttttgagtatattg
tagtttattttt**TG**tagttttttat**TG**ttattatggt**TG**t**TG**ggaat**TG**agg**TG**gttggg**TGTGTG**aggaagag
aagagaatt**TG**ggt**TG**tttaggttgtagaaagggg**TG**gg**TG**agagttaagaaggga**TG**tttgtagaagtggt**TG**t
aaatttggttttaagggtttt**TGTG**ggagagtttttttttttttgttttttttttttt**TG**tagttt**TGTG**gttat
atagtagt**TG**gag**TG**gttt**TG**gg**TG**tttagt**TG**gg**TG**g**TGTG**tagag**TG**gagggggagg**TG**t**TG**gtggaggagag
tgtgttta**TG**agagaggat**TGTG**gatttttt**TG**g**TG**a**TG**gttttt**TG**tgtattttgt**TG**gt**TGTGTG**gtttt
gt**TG**ttattgg**TG**ttag**TG**ttgttttgttta**TG**tttttt**TG**g**TG**tttag**TG**tattgtttagttagagtttttt
tt**TG**gtttagaggtttattaatgtttttt**TGTG**tttt**TG**gggagggggg**TG**ttaggtattattt**TG**ttaggttt**T**
**G**ttt**TG**ttttattt**TG**ggttt**TGTG**t**TG**ttttt**TG**ttttt**TGTG**ttgttttt**TG**ttttt**TGTG**ttgattttt**TG**t
tttttt**TG**ggttttttttttttattttttttt**TG**gagtttgaatggag**TGTG**gt**TG**ttggtttt**TG**gtta**TG**ggaa
tt**TG**a**TG**ttataaagag**TG**gagtaaataggg**TG**t**TG**ggtgtt**TG**ttattaggtttatttataaaat**TG**aggt**TG**a
gaggaagagggagg**TG**gtttt**TG**att**TG**t**TG**ttag**TG**g**TG**ggtag**TG**tt**TG**gattttgtgaatggag**TGTGTG**g**T**
**G**ttttt**TG**t**TG**tttatt**TGTG**ggtttagtttg**TG**t**TGTG**ttttagtt**TG**atat**TG**gtatttgtgt**TG**t**TG**ttgt
attt**TG**t**TG**ttagg**TG**gg**TG**g**TG**ttaaaggaaatagt**TG**gttgaggaggttagggtt**TG**ggtttgagtttttatt
ttttggtatttt**TGTG**tttttt**TGTG**tgatagttt**TG**taggttttttt**TG**gttatttaagtggagaggggat**TG**g
ag**TG**ttgttgtttatttag**TG**ggtttgatatttttagttttagattttt**TG**tg**TG**tttt**TG**gtt**TG**gttagtt
ttttttgaatatg**TG**attttgaga**TG**ttag**TG**tatgtattag**TG**tgat**TG**atttttatttaaagttattaaatt
agttttatagttttttttaagaa**TG**tgggtaatttttgtttggaagatgaaagagaaagtttttt**TG**tt**TG**aagt
ttaggat**TG**gagtgaggtgtggggtttgttgtttattatttaaaggaggtattaattgt**TG**gaatggtgttt**T**
**G**agggaggagagtaggttg**TG**ttttgttta**TG**ttttttaaaagatgtttatttaattttggt**TG**gggaaaaat
aaggtttt**TG**ggtttgggaaa**TG**ttttatttattttgtatagttgtgaattatttttttaggaaatgataggg
tttatatttgttg**TG**gagttggattttggaaaatgtatt**TG**taaagaatgatttttttgaaaggatttagtagtt
ttgggtt**TG**atttatgttttgggtattttttttttttttggtta**TG**tagtagtttttttttattttttattttttttt
tgtatttaatttttttagagg**TG**gttttggttggggggtttgtgta**TG**ta**TGTG**gtggggat**TG**tgtagaagttaa
aaattatt**TG**tt**TG**atatttttgttaagttttgttagtgagaattaattatagagtaagggagttataggatt**TG**t

tTGtagtttTGgaggttgtagagtagTGtattttatttTGgggTGTGTGtggaagttTGatttaggtagTGaggg
tatTGgtattTGtTGggtttttttTGtttaagtttatttttttttttatgttttgtttTGggTGgagTGgttttt
tTGagttttattttTGgtttTGgggttattttgtattttTGatTGgagttTGgTGgagttttattttTGa
gattttTGattggtttTGtttttattgggtttTGaatgtttTGattttTGggttttattTGgatgagttgtagg
ggtatTGtttgggtaaggtgatattttttttgtttTGggttttaaaatggtttaTGatggtattgtaTGgattt
ttttttttttaaatttagtatttatttattatggatttttgtattttttttttttaaagaggtattgta
ttaagatattttttttgattattgattttTGgTGtttttttaaatgttgTGtttaaggtttgtgtttattta
gtttaggtttggaaggagttataattgtggattgatTGaggttgttTGgtggtaggatttgggTGtTGggtat
ttTGggtgggagattagtggttaaagtTGtaTGgTGtggtttttttgtggggagggtaaaggtgtttgagttta
gtttttatttgttgaatgggattgtggggtttatttaataattTGttttTGataaggagtaaggagttttag
gggtttTGatagtttaaggttgatttgtttttTGggttgtttttagtagtttagaaattTGtTGtttgttag
gatttagggtTGgagtttttTGgTGtaagtaaatattgatttttttataaaggttttaataagatattttggg
aaaagtttttaggaggtaggttTGggttaatttaTGTGtgtTGtaggagttggggagaTGttttggggTGta
TGggtTGTGtttgggttagtttttgtttatttgttttggggtTGtttttttTGtTGgattTGTGtttggTGtt
taTGtggttagtaaataatagttaaatttagggggaggaaaatgtttatagaataaagaggtttgtttttgtat
ttgatagtaatTGttggtggagaagttttaaatttTGtaggTGatttataTGgaaaggggtgtgggagggTGTGt
gtgagtgtgtgtgtttaTGtgtgTGtgttTGTGtttaTGTGgttgtttattggaagggagttatagtttgggatt
taatgtgtttaagtTGagagttatttgaattgTGaaagTGaaggtttgatttttaaaaaagattaatttttaaaa
tttatatttattgttaatttaggaattggttttagttgtgtgaatttagtaaaggagatttaagttattata
ttgtatttatTGggatattgtgtaaatatttagatatatatttttttaattgtatgtttgtgaattattggttta
tagttaaatgagtataaTGTGagggttattttatgtTGgttatagataaaatttgttttaattTGtttgtagtt
tgtggaTGaaatataattatttaaataggaatttgtgaaatagtttgttaatgagtagatttggagaaaata
aaggtaatttggagtaagataTGgaatgttattttaaagttaatttaattaattaaggtatatttgtaggaattg
ggatgggattgtttaaggagaggtttttatttatatttagtagtaaatttTGggtgaaagaattttaaaaat
tTGttgtgataagtaaatttagtttttttttttattagttagtttttagatTGattttattagttattgttaggtt
ttagtaaaatatttgtttttgtttaggaatatttttttttaattaggaagttgtatgatTGgggaatttaaagtt
TGgagtagaattttagagagaagtatagttttatttagttttgatgttaattttgatttattgttttgttt
taaagtgtttgaataaaatttgtttgaaaaattattaatgaattaattaaggtggtgatggaaagaaaagttttg
tggtttttttttttttgagtaaagaggtagttttgttattaTGtTGaattggaataggaataatttattttgtat
agatttgttggttaggtttttagattaattaattagggaatttgtttttggtagtgggaaggtttaaaagaa
ttgggagatttttttttgtttttgaaagtagagaagtttgtggtttatatagTGtgtttttaggtaaTGtttaga
aatggaattgagaaatgtttgtttttttttaTGtattttgtttattaagttaggattgaaagTGaaggaaagtt
tttaaagtttatattgaatttaattaaaaattttaaagatatagtttgaggattttttttttttaagttttgatt
ttgttatagatttttttttgtttttatgtttgatatttagtatttattaatgatttaaaaggttatgtatTGgt
taggTGaggtggtttaTGttagtaattttagtattttggaagatTGaagtgggtagattatttgaggttaggagt
ttaagattagtttggttaatatggtgaaattttgtttttttttaaaaatataaaattagttagtatgaTGggTGTG
tatttataatttagttatattggaggttgaggtaggagaatTGtttgaattTGggaggtagaggttgtagtgag
ttgagatttttttattgtattttagtttgggTGataagagtaaaatttattttaaataaataaataaatataa
ataaaaaataataaaagattaagttttaatttagggttttgtagaaaggttttaatgtattgttttatttatt
gttttatttaaaattatttaatttaggttatgttattatttagtttggtggttttgggtaagatagttttttt
ggttttattttttttatttgtaaaatgaagagtttgggtaggatggtttttaggtttggtttattttTGatttt
gtatgattttgagtagaagaagatttttttagtttTGagtttaagttttataggtttttttatttTGtttttttgaag
agttaagtatattataatgttataagataaaggaTGattttagtttttttttttgtttttgttttagaatataaat
ttatggatagtagagtttatagttttatttatttgtttagttaTGtgttaaTGttttaagtattttgataaaaT
GtttttaaaatttagaaataaattttggaaagaaatatttttttaaaTGggataaaaatgaattTGgaggttgg
gtatggatggtggtttatgtttgtaatttttttagtattttgggaggttggTGgattatttgaggttaggagttgg
agattagtttgattaatattaaaaatataaaaattagttaggtatgatggtgggtgtttgtagttttagttattT
GtgaggttgaggtaaaagaatttttttgattTGgagaggtagaggttgtagtgagttaagattgggttattgtatt
tttgtttggatagggTGagatagTG

## Fig.6d

ggttaggatCGttaatgttaataaaCGgggtttCGttaggaCGgtgCGgaattgtaggtgatCGgagaaattCGt
tCGtattttttttgaggatttgaatttCGtatttgtatagaattttgtaaattttatatattaatatatataa
aggggCGCGCGgggattttCGtaattaCGaattaCGtCGgggattttttttCGtgttaataaggCGtaggtCGtt
aagatttttttatttttCGatttagttagCGttCGgagttttCGtttattttgagtaggtagatgtCGgtg
CGttCGgCGttgCGgCGtagCGtttgtattattagCGataCGgtgttgttttCGCGgaggttttCGgCGCGCGgg
ttgCGttCGtCGtagaCGatgatCGtCGagtagaggtCGgagCGtaagCGggCGCGtatttttttttCGgCGggt
aggatttgttttaggtttaCGgagtttttagttCGtCGtCGtaCGatggtgttatagCGtaCGttgatCGaattt
aggatgtagttCGCGttgtgCGttaggaaCGgtttgtagtttagtagtaggtatttgtCGtCGttCGgtagtttt
agggtgtCGtggttgtCGttgtCGttCGCGtCGtCGttattttCGtttCGgtttattagtttttgagtatattg

tagtttatttttCGtagtttttttatCGttattatggtCGtCGggaatCGaggCGgttgggCGCGCGaggaagag
aagagaattCGggtCGtttaggttgtagaaaggggCGggCGagagttaagaagggaCGtttgtagaagtggtCGt
aaatttggttttaagggttttCGCGggagagtttttttttttttgtttttttttttttCGtagtttCGCGgttat
atagtagtCGgagCGgtttCGggCGtttagtCGggCGgCGCGtagagCGgaggggaggCGtCGgtggaggagag
tgtgtttaCGagagaggatCGCGgatttttttCGgCGaCGgtttttCGtgtattttgtCGgtCGCGCGgtttt
gtCGttattggCGttagCGttgttttgtttaCGtttttCGgCGtttagCGtattgtttagttagagttttttt
ttCGgtttagaggtttattaatgtttttCGCGttttCGgggagggggCGttaggtattatttCGttaggtttC
GtttCGttttatttCGggtttCGCGtCGtttttCGtttttCGCGttgtttttCGtttttCGCGttgatttttCGt
ttttttCGggttttttttttttattttttttttCGgagtttgaatggagCGCGgtCGttggttttCGgttaCGggaa
ttCGaCGttataaagagCGgagtaaatagggCGtCGggtgttCGttattaggtttatttataaaatCGaggtCGa
gaggaagagggaggCGgttttCGattCGtCGttagCGgCGggtagCGttCGgatttgtgaatggagCGCGCGgC
GtttttCGtCGtttattCGCGggtttagtttgCGtCGCGttttagttCGatatCGgtatttgtgtCGtCGttgt
atttCGtCGttaggCGggCGgCGttaaaggaaatagtCGgttgaggaggttagggttCGggtttgagtttttatt
ttttggtattttCGCGtttttCGCGtgatagtttCGtaggtttttttCGgttatttaagtggagaggggatCGg
agCGttgttgtttatttagCGgggtttgatatttttttagtttagatttttCGtgCGttttCGgttCGgttagtt
ttttttgaatatgCGatttttgagaCGttagCGtatgtattagCGtgatCGatttttatttaaagttattaaatt
agttttatagtttttttaagaaCGtgggtaatttttttgtttggaagatgaaagagaaagtttttCGttCGaagt
ttaggatCGgagtgaggtgtggggtttgttgttttattatttaaagggaggtattaattgtCGgaatggtgtttC
GagggaggagagtaggttgCGtttttgtttaCGtttttttaaaagatgtttatttaattttggtCGggggaaaaat
aaggttttCGggtttgggaaaCGttttatttattttgtatagttgtgaattattttttttaggaaatgataggg
tttatatttgttgCGgagttggattttggaaaatgtattCGtaaagaatgatttttttgaaaggattttagtagtt
ttgggttCGatttatgtttttgggtattttttttttttttggttaCGtagtagtttttttattttttattttttttt
tgtatttaatttttttagaggCGgtttttggttggggggtttgtgtaCGtaCGCGgtggggatCGtgtagaagttaa
aaattattCGttCGatatttgttaagttttgttagtgagaattaattatagagtaagggagttataggattCGt
tCGtagtttCGgaggttgtagagtagCGtatttattttCGgggCGCGCGtggaagttCGatttaggtagCGaggg
tatCGgtattCGtCGggtttttttCGtttaagtttatttttttttttttatgtttttgtttCGggCGgagCGgtttt
tCGagttttttattttCGgtttCGgggggttattttttgtattttttCGatCGgagttCGgCGgagtttttattttCGa
gattttCGattggtttCGttttttattgggtttCGaatgtttCGattttCGggttttattCGgatgagttgtagg
ggtatCGtttgggtaaggtgatatttttttttgtttCGggttttaaaatggtttaCGatggtattgtaCGgattt
ttttttttttaaatttagtattttatttattatggatttttttgtattttttttttttttttaaagaggtattgta
ttaagatattttttttgattattgatttttCGgCGtttttttttaaatgttgCGtttaaggtttgtgtttatttta
gtttaggttttggaaggagttataattgtggattgatCGaggttgttCGgtggtaggatttgggCGtCGggtat
ttCGggtgggagattagtggttaaagtCGtaCGgCGtggtttttttttgtggggagggtaaaaggtgtttgagttta
gtttttttatttgttgaatggattgtgggggtttatttaataattCGttttCGaTAAGGAGTAAGGAGTTTTAG
GGGTTtCGatagtttaaggttgatttgtttttttCGggttgtttttttagtagtttagaaattCGtCGtttgttag
gatttagggtCGgagtttttCGgCGtaagtaaatattgattttttttataaaggttttaataagatattttttggg
aaaagtttttttaggaggtaggttCGggttaatttttaCGCGtgtCGtaggagttggggagaCGtttttggggCGta
CGggtCGCGtttgggttagttttgtttatttttgttttggggtCGtttttttttCGtCGgattCGCGtttggCGtt
taCGtggTTAGTAAATAATAGTTAAATTTAGGgggaggaaaatgtttatagaataaagaggtttgtttttttgtat
ttgatagtaatCGttggtggagaagttttaaatttCGtaggCGatttataCGgaaaggggtgtgggagggCGCGt
gtgagtgtgtgtgtttaCGtgtgCGtgttCGCGtttaCGCGgttgtttattggaagggagttatagtttgggatt
taatgtgtttaagtCGagagttatttgaattgCGaaagCGaaggtttgatttttaaaaaagattaatttttaaaa
tttatattttattgttaatttaggaattggtttttagttgtgtgaattttagtaaaggagatttaagttattata
ttgtatttatCGggatattgtgtaaatatttagatatatatttttttaattgtatgtttgtgaattattggttta
tagttaaatgagtataaCGCGagggttattttatgtCGgttatagataaaatttgtttttaattCGtttgtagtt
tgtggaCGaaatataattatttaaataggaattttgtgaaatagtttgttaatgagtagattttggagaaaata
aaggtaatttggagtaagataCGgaatgttattttaaagttaatttaattaattaaggtatatttgtaggaattg
ggatgggattgtttaaggagaggtttttttatttatatttagtagtaaatttCGgggtgaaagaatttttaaaaat
tCGttgtgataagtaaatttagtttttttttttattagttagtttttagatCGatttattagttattgttaggtt
ttagtaaaatatttgtttttgtttaggaatatttttttttttaattaggaagttgtatgatCGgggaatttaaagtt
CGgagtagaatttttagagagaagtatagtttttattttagttttgatgttaattttttgatttattgttttgttt
taaagtgtttgaataaaatttgtttgaaaaattattaatgaattaattaaggtggtgatggaaagaaaagttttg
tggttttttttttttttgagtaaagaggtagttttgttattaCGtCGaattggaataggaataatttattttgtat
agattttgttggttaggtttttttagattaattaattagggaatttgtttttttggtagtgggaaggtttaaaagaa
ttgggagattttttttttgtttttgaaagtagagaagtttgtggtttatatagCGtgtttttaggtaaCGtttaga
aatggaattgagaaatgtttgttttttttttaCGtatttttgtttattaagttaggattgaaagCGaaggaaagtt
tttaaagtttatattgaatttaattaaaaattttaaagatatagtttgaggatttttttttttttaagttttgatt
ttgttatagattttttttttgtttttttatgtttgatattttagtatttattaatgatttaaaaggttatgtatCGgt
taggCGaggtggtttaCGttagtaattttagtattttggaagatCGaagtgggtagattatttgaggttaggagt
ttaagattagtttggttaatatggtgaaatttttgtttttttttaaaaatataaaattagttagtatgaCGggCGCG

tatttataattttagttatattggaggttgaggtaggagaatCGtttgaattCGggaggtagaggttgtagtgag
ttgagatttttttattgtattttagtttgggCGataagagtaaaattttattttaaaataaataaataaatataa
ataaaaaataataaaagattaagttttaatttagggttttgtagaaaggttttaatgtattgttttatttatt
gttttattttaaaattatttaatttaggttatgttattatttagtttggtggttttgggtaagatagttttttt
ggttttattttttttatttgtaaaatgaagagtttgggtaggatggtttttaggtttggtttattttCGatttt
gtatgattttgagtagaagaagatttttagtttCGagtttaagttttataggtttttatttCGtttttgaag
agttaagtatattataatgttataagataaaggaCGattttagtttttttttgttttgttttagaatataaat
ttatggatagtagagtttatagtttttatttatttgtttagttaCGtgttaaCGttttaagtattttgataaaaC
GtttttaaaatttagaaataaatttggaaagaaatattttttaaaCGggataaaaatgaattCGgaggttgg
gtatggatggtggtttatgtttgtaatttttagtattttgggaggttggCGgattatttgaggttaggagttgg
agattagtttgattaatattaaaaatataaaaattagttaggtatgatggtgggtgtttgtagttttagttattC
GtgaggttgaggtaaaagaatttttgattCGgagaggtagaggttgtagtgagttaagattgggttattgtatt
tttgtttggatagggCGagatagCG

## Fig.6e

ggttaggatc(Sau3AI)GttaatgttaataaaCGgggtttCGttaggaCGgtgCGg(AciI)aattgtaggtga
tc(Sau3AI)GgagaaattCGttCGtatttttttgaggatttgaatttCGtatttgtatagaaattttgtaaat
ttttatatattaatatatataaaggggCGc(HinP1I)(HhaI)g(BstUI)c(BssHII)gg(AciI)ggattt
tCGtaattaCGaattaCGt(TaiI)CGgggatttttttCGtgttaataaggCGtaggtCGttaagattttttt
atttttCGa(TaqI)ttttagttagCGttCGgagttttCGtttattttgagtaggtagatgtCGgtgCGttCG
gCGttgCGg(AciI)CGtagCGtttgtattattagCGataCGgtgttgttttCGCG(BstUI)g(AciI)aggtt
ttCGgCGc(HinP1I)(HhaI)g(BstUI)c(BssHII)gg(AciI)gttgCGttCGtCGtagac(AccI)Gatg
atc(Sau3AI)GtCGa(TaqI)gtagaggtCGgagCGtaagCGg(AciI)gCGc(HinP1I)(HhaI)G(BSTUI
)tattttttttCGgCGg(AciI)gtaggatttgttttaggtttaCGgagttttagttCGtCGtCGtac(RsaI
)(Csp6I)g(BsiWI)atggtgttatagCGtac(RsaI)(Csp6I)g(BsiWI)ttgatc(Sau3AI)Gaattta
ggatgtagttCGCG(BSTUI)ttgtgCGttaggaaCGgtttgtagtttagtagtaggtatttgtCGtCGttCGgt
agttttagggtgtCGtggttgtCGttgtCGttCGCG(BSTUI)tCGtCGttattttCGtttCGgtttattagttt
tttgagtatattgtagtttatttttCGtagtttttttatCGttattatggtCGtCGggaatc(TfiI)ga(TaqI
)ggCGg(AciI)ttgggCGc(HinP1I)(HhaI)g(BstUI)c(BssHII)Gaggaagagaagagaattc(EcoR
I)GggtCGtttaggttgtagaaaggggCGg(AciI)gCGagagttaagaaggaCGt(TaiI)ttgtagaagtgg
tCGtaaatttggtttttaaggttttCGCG(BstUI)g(AciI)gagagttttttttttttttgtttttttttttt
CGtagtttCGCG(BstUI)g(AciI)ttatatagtagtCGgagCGg(BsrBI)(AciI)tttCGggCGtttagtC
GggCGg(AciI)CGCG(BSTUI)tagagCGg(BsrBI)(AciI)aggggaggCGtc(BsaHI)(AhaII)Ggtg
gaggagagtgtgtttaCGagagaggatc(Sau3AI)gCG(BstUI)g(AciI)atttttttCGgCGaCGgttttt
CGtgtattttgtCGgtCG(BsiEI)CGCG(BstUI)g(AciI)tttttgtCGttattggCGttagCGttgtttt
gtttaCGt(TaiI)tttttCGgCGtttagCGtattgttttagttagagttttttttCGgtttagaggtttatta
atgtttttCGCG(BSTUI)ttttCGggggagggggCGttaggtattatttCGttaggtttCGtttCGttttatt
tCGggtttCGCG(BSTUI)tCGtttttCGtttttCGCG(BSTUI)ttgtttttCGtttttCGCG(BSTUI)ttga
tttttCGtttttttCGggtttttttttttattttttttttCGgagtttgaatggagCGc(HinP1I)(HhaI)g(B
stUI)g(AciI)tCGttggttttCGgttaCGggaattc(EcoRI)GaCGt(TaiI)tataaagagCGg(BsrBI)
(AciI)agtaaatagggCGtc(BsaHI)(AhaII)GggtgttCGttattaggtttatttataaaatCGa(TaqI)
ggtCGa(TaqI)gaggaagagggaggCGg(AciI)ttttCGa(TaqI)ttCGtCGttagCGg(AciI)CGggtag
CGttCGgatttgtgaatggagCGc(HinP1I)(HhaI)g(BstUI)c(BssHII)gg(AciI)CGtttttCGtC
GtttattCGCG(BstUI)g(AciI)gtttagtttgCGtCGCG(BSTUI)tttttagttCGa(TaqI)tatc(Eco
RV)GgtatttgtgtCGtCGttgtatttCGtCGttaggCGg(AciI)gCGg(AciI)CGttaaaggaaatagtCGg
ttgaggaggttagggttCGggtttgagttttatttttggtattttCGCG(BSTUI)ttttttCGCG(BSTUI)
tgatagtttCGtaggtttttttCGgttatttaagtggagaggggatc(Sau3AI)GgagCGttgttgtttattta
gCGg(AciI)ggtttgatatttttagttttagattttCGtgCGttttCGgttCGgttagttttttttgaatat
gCGattttgagaCGt(TaiI)tagCGtatgtattagCGtgatc(Sau3AI)gat(ClaI)(BspDI)ttttatt
taaagttattaaattagttttatagttttttaagaaCGt(TaiI)gggtaatttttgtttggaagatgaaaga
gaaagtttttCGttCGa(TaqI)a(BstBI)gtttaggatc(Sau3AI)Ggagtgaggtgtggggtttgttgtt
ttattatttaaagggaggtattaattgtCGgaatggtgtttCGa(TaqI)gggaggagagtaggttgCGttttg
tttaCGt(TaiI)tttttaaaagatgtttatttaattttggtCGgggaaaaataaggttttCGggtttgggaaa
CGt(TaiI)t(Psp1406I)tttatttatttgtatagttgtgaattatttttttaggaaatgatagggtttat
atttgttgCGg(AciI)agttggattttggaaaatgtattCGtaaagaatgatttttgaaaggattttagtagt
tttgggttCGa(TaqI)tttatgttttgggtatttttttttttggttaCGt(TaiI)a(BsaAI)gtagtttt
ttttatttttatttttttttgtatttaatttttagaggCGg(AciI)tttttggttggggtttgtgtac(Rs
aI)(Csp6I)gta(BsaAI)CGCG(BstUI)g(AciI)tggggatc(Sau3AI)Gtgtagaagttaaaaattatt
CGttCGa(TaqI)tatttgttaagttttgttagtgagaattaattatagagtaagggagttataggattc(Tfi
I)GttCGtagtttCGgaggttgtagagtagCGtattttatttCGgggCGc(HinP1I)(HhaI)g(BstUI)c(B

ssHII)Gtggaagtt**CG**a(TaqI)tttaggtag**CG**agggtat**CG**gtatt**CG**t**CG**ggttttttt**CG**tttaagttta
ttttttttttatgttttgttt**CG**gg**CG**g(AciI)ag**CG**g(AciI)tttttt**CG**a(TaqI)gttttatttt**CG**g
ttt**CG**gggttatttttgtattttt**CG**a(TaqI)tc(Sau3AI)g(PvuI)(BsiEI)gagtt**CG**g**CG**g(AciI)
agttttatttt**CG**a(TaqI)gatttt**CG**a(TaqI)ttggttt**CG**tttttattgggttt**CG**a(TaqI)a(BstB
I)tgttt**CG**a(TaqI)tttt**CG**ggttttatt**CG**gatgagttgtaggggtat**CG**tttgggtaaggtgatattttt
tttgttt**CG**ggttttaaaatggttta**CG**atggtattgtac(RsaI)(Csp6I)Ggatttttttttttttaaaat
ttagtatttatttattatggatttttgtatttttttttttttaaagaggtattgtattaagatattttttt
gattattgattttt**CG**g**CG**tttttttaaatgttg**CG**tttaaggtttgtgtttattttagtttaggttttggaag
gagttataattgtggattgatc(Sau3AI)Gaggttgtt**CG**gtggtaggattttggg**CG**tc(BsaHI)(AhaII)
Gggtattt**CG**ggtgggagattagtggttaaagt**CG**tac(RsaI)(Csp6I)g(BsiWI)g**CG**tggtttttttgtg
gggagggtaaaaggtgtttgagtttagtttttttatttgttgaatgggattgtgggggtttatttaataatt**CG**t
ttt**CG**a(TaqI)taaggagtaaggagttttaggggttt**CG**a(TaqI)tagtttaaggttgattttgtttttt**CG**g
gttgtttttttagtagtttagaaatt**CG**t**CG**tttgttaggatttagggt**CG**gagtttttt**CG**g**CG**taagtaaatat
tgatttttttataaaggttttaataagatattttgggaaaagtttttttaggaggtaggtt**CG**ggttaatttta**C**
**GCG**(BSTUI)tgt**CG**taggagttggggaga**CG**t(TaiI)ttttgggg**CG**tac(RsaI)(Csp6I)g(BsiWI)gg
t**CGCG**(BSTUI)ttttgggttagtttttgtttatttttgttttggggt**CG**ttttttt**CG**t**CG**gattc(TfiI)G**CG**
(BSTUI)tttgg**CG**ttta**CG**t(TaiI)g(BsaAI)gttagtaaataatagttaaatttaggggggaggaaaatgtt
tatagaataaagaggtttgtttttttgtatttgatagtaat**CG**ttggtggagaagttttaaattt**CG**tagg**CG**att
tata**CG**gaaaggggtgtgggaggg**CG**c(HinP1I)(HhaI)G(BSTUI)tgtgagtgtgtgtgttta**CG**t(TaiI
)g(BsaAI)tg**CG**tgtt**CGCG**(BSTUI)ttta**CGCG**(BstUI)g(AciI)ttgtttattggaagggagttatagt
ttgggatttaatgtgtttaagt**CG**a(TaqI)gagttatttgaattg**CG**aaag**CG**aaggtttgattttaaaaaag
attaattttaaaatttatatttattgttaatttaggaattggtttttagttgtgtgaattttagtaaaggaga
tttaagttattatattgtatttat**CG**ggatattgtgtaaatatttagatatatatttttttaattgtatgtttgt
gaattattggtttatagttaaatgagtataa**CGCG**(BSTUI)agggttattttatgt**CG**gttatagataaaattt
gtttttaatt**CG**tttgtagtttgtgga**CG**aaatataattattttaaataggaattttgtgaaatagtttgttaat
gagtagatttggagaaaataaaggtaatttggagtaagata**CG**gaatgttattttaaagttaatttaattaatt
aaggtatatttgtaggaattgggatgggattgtttaaggagaggttttttatttatatttagtagtaaattt**CG**g
ggtgaaagaattttttaaaaatt**CG**ttgtgataagtaaatttagtttttttttttattagttagttttttagatc(Sa
u3AI)gat(ClaI)(BspDI)tttattagttattgttaggttttagtaaaatatttgtttttgtttaggaatatt
ttttttaattaggaagttgtatgatc(Sau3AI)Ggggaatttaaagtt**CG**gagtagaattttttagagagaagt
atagttttatttttagttttgatgttaatttttgatttattgttttgttttaaagtgtttgaataaaatttgttt
gaaaaattattaatgaattaattaaggtggtgatggaaagaaaagttttgtggtttttttttttttgagtaaaga
ggtagttttgttatta**CG**t(TaiI)**CG**aattggaataggaataatttatttgtatagattttgttggttaggtt
ttttagattaattaattagggaatttgtttttttggtagtgggaaggtttaaaagaattgggagatttttttttgt
ttttgaaagtagagaagtttgtggtttatatag**CG**tgtttttaggtaa**CG**t(TaiI)t(Psp1406I)tagaaat
ggaattgagaaatgtttgtttttttttta**CG**t(TaiI)a(BsaAI)tttttgtttattaagttaggattgaaag**CG**
aaggaaagttttttaaagtttatattgaatttaattaaaaattttaaagatatagtttgaggattttttttttta
agttttgattttgttatagattttttttgtttttttatgtttgatattttagtatttattaatgatttaaaaggtt
atgtat**CG**gttagg**CG**aggtggttta**CG**t(TaiI)tagtaattttagtattttggaagatc(Sau3AI)Gaagtg
ggtagattatttgaggttaggagtttaagattagtttggttaatatggtgaaattttgttttttttaaaaatata
aaattagttagtatga**CG**gg**CG**c(HinP1I)(HhaI)G(BSTUI)tatttataattttagttatattggaggttg
aggtaggagaatc(TfiI)Gtttgaattc(EcoRI)Gggaggtagaggttgtagtgagttgagattttttttattg
tattttagtttggg**CG**ataagagtaaaatttttatttttaaaataaataaataaatataaataaaaaataataaaag
attaagttttttaatttagggtttttgtagaaaggttttaatgtattgtttttatttattgttttattttaaaatta
tttaatttaggttatgttattatttagtttggtggttttgggtaagatagtttttttttggtttttattttttttat
ttgtaaaatgaagagtttgggtaggatggtttttttaggtttggtttatttt**CG**a(TaqI)ttttgtatgattttg
agtagaagaagatttttttagttt**CG**a(TaqI)gtttaagttttataggtttttttattt**CG**tttttttgaagagtta
agtatattataatgttataagataaagga**CG**atttttagtttttttttttgtttttgttttagaatataaatttatg
gatagtagagtttatagtttttatttatttgtttagtta**CG**t(TaiI)g(BsaAI)ttaa**CG**t(TaiI)t(Psp1
406I)ttaagtattttgataaaa**CG**t(TaiI)t(Psp1406I)tttaaaattttagaaataaattttggaaagaa
atatttttttaaa**CG**gataaaaatgaattc(EcoRI)Ggaggttgggtatggatggtggtttatgtttgtaatt
ttttagtattttgggaggttgg**CG**g(AciI)attatttgaggttaggagttggagattagtttgattaatattaa
aaatataaaaaattagttaggtatgatggtgggtgtttgtagttttagttatt**CG**tgaggttgaggtaaaagaatt
ttttgattc(TfiI)Ggagaggtagaggttgtagtgagttaagattgggttattgtatttttgtttggatagggg**C**
**G**agatag**CG**

European Patent Office

# EUROPEAN SEARCH REPORT

Application Number
EP 07 02 1849

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/68911 A (EPIGENOMICS AG [DE]; OLEK ALEXANDER [DE]; PIEPENBROCK CHRISTIAN [DE];) 20 September 2001 (2001-09-20)<br>* page 8, paragraph 3 *<br>* page 16, paragraph 2 *<br>* claims 1,3-9,29,32 *<br>-& DATABASE EMBL [Online]<br>5 October 2001 (2001-10-05), "Sequence 97 from Patent WO0168911."<br>XP002472975<br>retrieved from EBI accession no. EMBL:AX251836<br>Database accession no. AX251836<br>-& DATABASE EMBL [Online]<br>5 October 2001 (2001-10-05), "Sequence 98 from Patent WO0168911."<br>XP002472976<br>retrieved from EBI accession no. EMBL:AX251837<br>Database accession no. AX251837<br>----- | 16,17 | INV.<br>C12Q1/68 |
| X<br><br><br>Y | WO 03/076594 A (UNIV JOHNS HOPKINS [US]; SIDRANSKY DAVID [US] UNIV JOHNS HOPKINS [US];) 18 September 2003 (2003-09-18)<br>* table 6 *<br>* claims 17,19,21-24,29,33,34,40 *<br>----- | 1,3-6,<br>13,15<br><br>7-12 | TECHNICAL FIELDS SEARCHED (IPC)<br>C12Q |
| X | WO 2007/016668 A (SEQUENOM INC [US]; VAN DEN BOOM DIRK JOHANNES [US]; EHRICH MATHIAS [US) 8 February 2007 (2007-02-08)<br>* page 92 *<br>* page 116 - page 118 *<br>* claims 17,18 *<br>-----<br>-/-- | 1,3-6,<br>13-17 | |

The present search report has been drawn up for all claims

| Place of search | Date of completion of the search | Examiner |
|---|---|---|
| Munich | 14 March 2008 | Schmitt-Humbert, C |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 07 02 1849

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PAZ MARIA F ET AL: "CpG island hypermethylation of the DNA repair enzyme methyltransferase predicts response to temozolomide in primary gliomas." CLINICAL CANCER RESEARCH : AN OFFICIAL JOURNAL OF THE AMERICAN ASSOCIATION FOR CANCER RESEARCH 1 AUG 2004, vol. 10, no. 15, 1 August 2004 (2004-08-01), pages 4933-4938, XP002419891 ISSN: 1078-0432 * abstract * ----- | 2,14 | |
| Y | TOST J ET AL: "ANALYSIS AND QUANTIFICATION OF MULTIPLE METHYLATION VARIABLE POSITIONS IN CPG ISLANDS BY PYROSEQUENCING" BIOTECHNIQUES, INFORMA LIFE SCIENCES PUBLISHING, WESTBOROUGH, MA, US, vol. 35, no. 1, July 2003 (2003-07), pages 152-154,156, XP001156833 ISSN: 0736-6205 * the whole document * ----- | 7-9 | TECHNICAL FIELDS SEARCHED (IPC) |
| Y | XIONG Z AND LAIRD P W: "COBRA: a sensitive and quantitative DNA methylation assay" NUCLEIC ACIDS RESEARCH, OXFORD UNIVERSITY PRESS, SURREY, GB, vol. 25, no. 12, 1997, pages 2532-2534, XP002106407 ISSN: 0305-1048 * the whole document * ----- -/-- | 7,10-12 | |

The present search report has been drawn up for all claims

| Place of search | Date of completion of the search | Examiner |
|---|---|---|
| Munich | 14 March 2008 | Schmitt-Humbert, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

European Patent Office

# EUROPEAN SEARCH REPORT

Application Number
EP 07 02 1849

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ESTECIO MARCOS R H ET AL: "High-throughput methylation profiling by MCA coupled to CpG island microarray" GENOME RESEARCH, COLD SPRING HARBOR LABORATORY PRESS, WOODBURY, US, vol. 17, no. 10, October 2007 (2007-10), pages 1529-1536, XP009097041 ISSN: 1088-9051 * abstract * * figure 1 * *Methods* * page 1531, column 1, last paragraph - column 2, paragraph 1 * ----- | 1,3-13, 15-17 | |
| A | LIM S ET AL.: "DUSP7 and DUSP8 promoter hypermethylations: predictors of clinical outcomes in advanced epithelial ovarian carcinoma." JOURNAL OF CLINICAL ONCOLOGY, 2007 ASCO ANNUAL MEETING PROCEEDINGS, ABSTRACT 5501, vol. 25, no. 18S, 20 June 2007 (2007-06-20), XP002472973 * the whole document * ----- | 1,3-13, 15-17 | TECHNICAL FIELDS SEARCHED (IPC) |
| T | WAHA A ET AL.: "Glio-methyl-predictive epigenetic parameter for gliomas" 1 February 2008 (2008-02-01), XP002472974 Retrieved from the Internet: URL:http://www.biovaria.org/downloads/BioVaria_P5.pdf> * the whole document * ----- | 1-17 | |

The present search report has been drawn up for all claims

2

| Place of search | Date of completion of the search | Examiner |
|---|---|---|
| Munich | 14 March 2008 | Schmitt-Humbert, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 07 02 1849

This annex lists the patent family members relating to the patent documents cited in the above-mentioned European search report.
The members are as contained in the European Patent Office EDP file on
The European Patent Office is in no way liable for these particulars which are merely given for the purpose of information.

14-03-2008

| Patent document cited in search report | Publication date | Patent family member(s) | Publication date |
|---|---|---|---|
| WO 0168911 A | 20-09-2001 | AU 4835201 A | 24-09-2001 |
| | | AU 5038101 A | 24-09-2001 |
| | | WO 0168912 A2 | 20-09-2001 |
| | | EP 1283905 A2 | 19-02-2003 |
| | | EP 1268855 A2 | 02-01-2003 |
| | | JP 2004507213 T | 11-03-2004 |
| | | JP 2004507214 T | 11-03-2004 |
| | | US 2004029123 A1 | 12-02-2004 |
| | | US 2004048254 A1 | 11-03-2004 |
| WO 03076594 A | 18-09-2003 | AU 2003230616 A1 | 22-09-2003 |
| | | CA 2478510 A1 | 18-09-2003 |
| | | CN 101080499 A | 28-11-2007 |
| | | EP 1578924 A2 | 28-09-2005 |
| | | JP 2005532041 T | 27-10-2005 |
| WO 2007016668 A | 08-02-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GARDINER-GARDEN, M. ; FROMMER, M.** *J. Mol. Biol.,* 1987, vol. 196 (2), 261-282
- **PALMISANO, WA et al.** *Cancer Res.,* 2000, vol. 60, 5954-5958
- **HERMAN, J.G. et al.** *Proc. Natl. Acad. Sci. USA,* 1986, vol. 93, 9821-26
- **DERKS, S. et al.** *M. Cell Oncol.,* 2004, vol. 26, 291-99
- **HEGI et al.** *N. Engl. J. Med.,* 2005, vol. 352, 997-1003
- **SHAW, R.J. et al.** *Nucl. Acids Res.,* 2006, vol. 34, e78
- **SAM-BROOK et al.** Molecular Cloning: A Laboratory Manual. Cold Spring Harbor Laboratory Press, 2001
- Current Protocols in Molecular Biology, Current Protocols in Immunology, Current Protocols. **AUSUBEL et al.** Protein Science, and Current Protocols in Cell Biology. Wiley Interscience, 1987